# EUROPEAN PATENT APPLICATION

(11) **EP 2 901 936 A1**
(43) Date of publication of application: **05.08.2015**
(21) Application number: 13841063.4
(22) Date of filing: 24.07.2013
(51) Int. Cl.: A61B 8/00

(54) **PORTABLE ULTRASONIC IMAGING DEVICE**

(30) Priority: 28.09.2012 JP 2012217313; 28.09.2012 JP 2012217312
(71) Applicant: Hitachi Aloka Medical, Ltd., Tokyo 181-8622 (JP)
(72) Inventor: NINOMIYA, Atsushi, Chiyoda-ku, Tokyo 100-8280 (JP); YANASE, Kazuyuki, Chiyoda-ku, Tokyo 100-8280 (JP); YOKOYAMA, Masaru, Chiyoda-ku, Tokyo 100-8280 (JP); USAMI, Katsumi, Chiyoda-ku, Tokyo 100-8280 (JP); KASANAMI, Tsuneo, Mitaka-shi, Tokyo 181-8622 (JP)
(74) Representative: Strehl Schübel-Hopf & Partner
(86) International application number: PCT/JP2013/070093
(87) International publication number: WO 2014/050279

(57) **Abstract**

A portable ultrasound imaging apparatus is provided, being operable when used in hand-held mode, and capable of performing operations such as starting and ending actions easily, even during an examination. The portable ultrasound imaging apparatus has a display panel 30 and an operation panel 20 on the front surface of a main body 10 that incorporates an electronic circuit constituting an ultrasound imager, and a handle on the back surface, and the apparatus is provided with operating portions 81 and 82 on the back surface, separated from the operation panel 20. The operating portion 81 may be provided on the handle 60, for instance, and while performing examination with holding the apparatus main body via the handle 60, the operating portion on the back surface is manipulated to implement functions such as starting and ending the imaging.

## Description

### Technical Field

The present invention relates to a portable ultrasonic imaging device, and in particular, it relates to a portable ultrasonic imaging device that allows manipulation while holding the apparatus by a single hand.

### Background Art

An ultrasonic imaging apparatus is widely used as a medical image diagnostic apparatus, relatively simple and non-invasively applicable, and various types of portable ultrasonic diagnostic apparatus have been developed, being available not only in a laboratory within a hospital, but also in a hospital room or out of hospital. By way of example, a notebook ultrasound diagnostic apparatus is put into practical use, having a structure that couples a display panel with an operation panel at one end in an openable and closable manner, the display panel being configured to display an image, and the like, taken by the ultrasonic diagnostic apparatus, and the operation panel being configured to input instructions necessary for ultrasonic examination. In addition, an ultrasound diagnostic apparatus being configured in such a manner that a main body provided with the operation panel is coupled to the display panel via hinges is also put into practical use (e.g., Patent Document 1, etc.).

In general, a usage pattern of those kinds of portable ultrasound diagnostic apparatus is to place the apparatus on a table such as a desk. In the case of the aforementioned notebook ultrasound diagnostic apparatus, the main body is placed on the table in such a manner that the operation panel faces upward, and the display panel is opened. Then, an ultrasound probe connected to the main body is applied to an examination region of a patient, and the examination is conducted while manipulating the operation panel, along with checking an ultrasound image shown on the screen of the display panel.

In contrast to the aforementioned portable ultrasound diagnostic apparatus to be placed on a table for manipulation, there is suggested a handheld portable ultrasound diagnostic apparatus that is operable while being held by a single hand (Patent Documents 2 and 3).

### Prior Art Document

### Patent Document

Patent Document 1 Japanese Unexamined Patent Application Publication No. 2010-162107
Patent Document 2 Japanese Unexamined Patent Application Publication No. 2010-131396
Patent Document 3 Japanese Unexamined Patent Application Publication No. 2012-50516

### Disclosure of the Invention

### Problem to be solved by the Invention

The portable ultrasound diagnostic apparatus as described in the Patent Documents 2 and 3 is manipulated in an examination, holding the apparatus in one hand and the probe in the other hand. In this case, the examination with holding the apparatus in one hand requires that the apparatus is light in weight and small in size to some extent. Even though it is a portable type, the main body of the apparatus needs to have a certain size and weight so as to have an independent ultrasound measurement function in itself. Accordingly, the manipulation in the state of being held in one hand may become unstable. In addition, both hands are occupied during the examination, and when the need for manipulating the operation panel arises, the probe may be placed on the table, for instance, and the operating panel is manipulated by the hand having held the probe, suspending the examination on the way.

The present invention is directed to providing a portable ultrasound imaging apparatus that is operable while holding the apparatus by a single hand, allowing a stable posture when used in an examination, and being operable by the hand that holds the apparatus even while the examination is performed.

### Means to solve the Problem

In order to solve the problem above, the portable ultrasound imaging apparatus of the present invention is provided with a main body incorporating an electronic circuit constituting an ultrasound imager, and when one surface of the main body is assumed as a front surface and the surface on the other side of the front surface is assumed as a back surface, the apparatus is characterized as having a display panel configured to display an image on the front surface, and an operating portion on the back surface.

### Effect of the Invention

The portable ultrasound imaging apparatus of the present invention allows a handle of the apparatus main body to be held by one hand, and when an ultrasound examination is performed with the other hand manipulating a probe, or the like, the hand that holds the handle is enabled to manipulate the apparatus easily.

### Brief Description of Drawings

FIG. 1 is an overall view of one embodiment of the portable ultrasound imaging apparatus of the present invention, illustrating the apparatus in perspective, viewed from the front side;
FIG. 2 illustrates in perspective the back-right side and the back-left side of the portable ultrasound imaging apparatus of the FIG. 1;
FIG. 3 illustrates six views (the front view, left side view, right side view, plan view, and bottom view, except the rear view) of the portable ultrasound imaging apparatus as shown in FIG. 1;
FIG. 4 is a functional block diagram illustrating the configuration of the ultrasound imager;
FIG. 5 illustrates an overall view of the probe that is generally used for the ultrasound imaging apparatus;
FIG. 6 illustrates an example of the operation button provided on the handle, FIG. 6(a) is a plan view of the handle, FIG. 6(b) is a top view, and FIG. 6(c) illustrates a usage pattern;
FIG. 7 illustrates one example where the operation buttons are provided on the back surface of the main body;
FIG. 8 illustrates a usage example of the operation buttons as shown in FIG. 7;
FIG. 9 is a front-side perspective view showing a mechanism for mounting the operation panel;
FIG. 10 is a back-side perspective view showing a mechanism for mounting the operation panel;
FIG. 11 illustrates an essential portion of the mounting mechanism as shown in FIG. 10;
FIG. 12 is a perspective view showing a component of a mechanism for mounting the operation panel;
FIG. 13 is a perspective view showing a structure of the operation panel side;
FIG. 14 illustrates variation of the posture of the operation panel; FIG. 14(a) illustrates the initial state, FIG. 14 (b) illustrates the slide state sliding to the lowermost end, FIG. 14 (c) illustrates the slide state being moved to a desired slide position, and FIG. 14 (d) illustrates the state tilted at the position of FIG. 14(c);
FIG. 15 illustrates an example how to use the operation panel according to the usage state of the apparatus;
FIG. 15 (a) illustrates the state that an operator holds the apparatus, and FIG. 15(b) illustrates the state that the apparatus is placed on the table;
FIG. 16(a), FIG. 16(b), and FIG. 16(c) illustrate modification example of the mounting mechanism of the operation panel and posture variation thereof;
FIG. 17 is a rear view of the portable ultrasound imaging apparatus as shown in FIG. 1;
FIG. 18 is a cross-sectional view of a mounting mechanism of the handle and a stand portion;
FIG. 19 is a perspective view showing the mounting mechanism of the handle;
FIG. 20 is a perspective view showing the mounting mechanism of the stand portion;
FIG. 21 is a perspective view showing the essential portion of the mounting mechanism of the handle and the stand portion;
FIG. 22(a) and FIG. 22(b) illustrate different usage states of the stand portion;
FIG. 23(a) and FIG. 23(b) illustrate different usage states of the handle;
FIG. 24 illustrates a back side panel and a back side cover of the portable ultrasound imaging apparatus;
FIG. 25 illustrates only the essential portion of the cross sectional view taken along line A-A of FIG. 24;
FIG. 26(a) and FIG. 26(b) respectively illustrate the right side view and the left side view of FIG. 24;
FIG. 27 is a perspective view showing a connector of the probe;
FIG. 28(a), FIG. 28(b), and FIG. 28 (c) illustrate other embodiments of the back side panel;
FIG. 29 illustrates an embodiment of a wireless probe;
FIG. 30 is a functional block diagram showing the probe shown in FIG. 29 and the main body;
FIG. 31 illustrates a usage pattern of the probe shown in FIG. 29;
FIG. 32 illustrates another usage pattern of the probe as shown in FIG. 29;
FIG. 33 illustrates an embodiment of another wireless probe;
FIG. 34(a) and FIG. 34(b) are a cross-sectional view and a plan view, respectively, illustrating a structure of the operation buttons;
FIG. 35(a), FIG. 35(b), and FIG. 35(c) illustrate the operation buttons, different types in shape;
FIG. 36 is a functional block diagram showing functions of the controller 43 configured to control GUI;
FIG. 37 is a flow diagram showing one example of a control procedure;
FIG. 38 illustrates one example of a display screen when measurement starts;
FIG. 39 illustrates one example of assignment of functions to the operation buttons;
FIG. 40 is a flow diagram showing a modification example of the control procedure;
FIG. 41 illustrates the portable ultrasound imaging apparatus provided with a probe holder on the back surface, illustrating the main body in perspective, viewed from the back side;
FIG. 42 is a cross-sectional view of the portable ultrasound imaging apparatus as shown in FIG. 41; and
FIG. 43 is a perspective view showing the probe holder and a cup, when the cup is removed from the portable ultrasound imaging apparatus of FIG. 41.

### Best Mode for Carrying Out the Invention

Hereinafter, with reference to the accompanying drawings, an explanation will be provided as to an embodiment of the portable ultrasound imaging apparatus according to the present invention. The portable ultrasound imaging apparatus of the present embodiment includes a main body (10) incorporating an electronic circuit constituting an ultrasound imager (40), and when one surface of the main body is assumed as a front surface and the surface on the other side of the front surface is assumed as a back surface, the apparatus is characterized as having a display panel (30) configured to display an image on the front surface, and an operating portion (81, 82) on the back surface. In addition, the portable ultrasound imaging apparatus of the present embodiment is provided with a handle (60) on the back surface, and the handle (60) is equipped with the operating portion (81). Alternatively, the operating portion (82) may be provided in proximity to the handle (60), on the back surface of the main body.

The portable ultrasound imaging apparatus of the present embodiment may further be provided with an operation panel (20) in parallel with the display panel (30), on the front surface side of the main body (1). This operation panel (20) on the front surface side is capable of functioning as an input unit for the ultrasound imager (40), together with the operating portion on the back side.

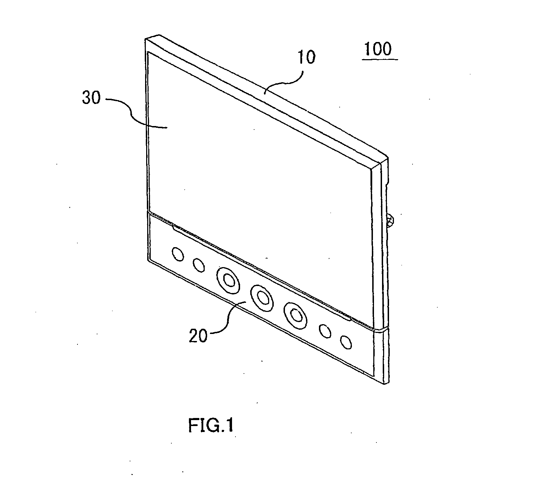

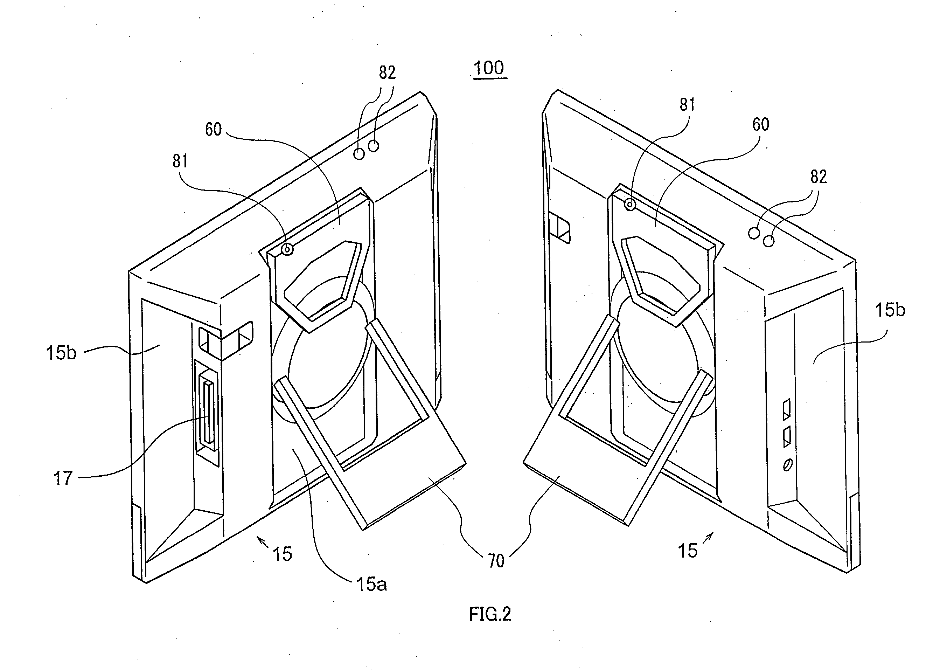

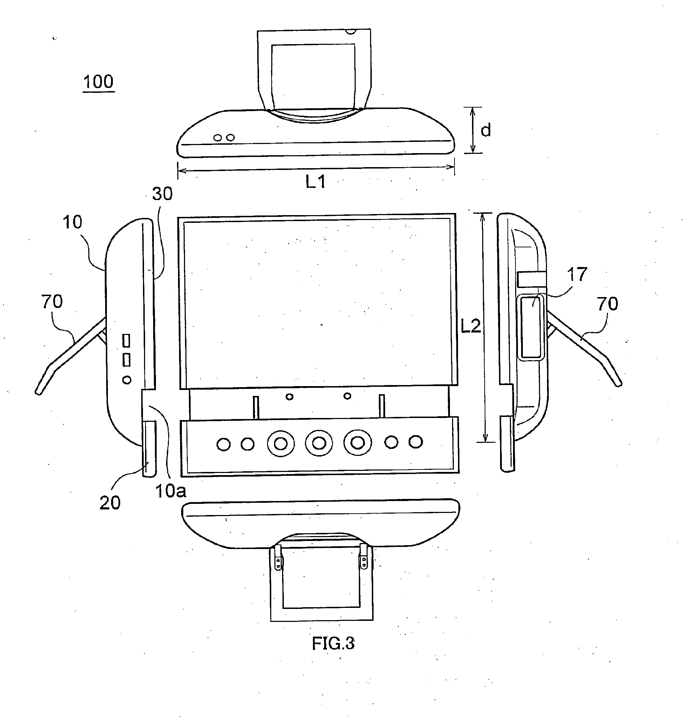

Firstly, with reference to the drawings from FIG. 1 to FIG. 3, an overview of the portable ultrasound imaging apparatus of the present embodiment will be explained. FIG. 1 is a perspective view showing the overall portable ultrasound imaging apparatus of the present embodiment, viewed from the front side, FIG. 2 illustrates perspective views of the portable ultrasound imaging apparatus viewed from the back side, and FIG. 3 illustrates six views (the front view, left side view, right side view, plan view, and bottom view, except the rear view) of the portable ultrasound imaging apparatus as shown in FIG. 1, with the operation panel that is made to slide.

The portable ultrasound imaging apparatus 100 of the present embodiment is a notebook or tablet device, with the thickness d, being thin relative to the horizontal size L1 and the vertical size L2, provided with the main body 10 incorporating an electronic circuit, and the like, necessary for ultrasound examination, and the operation panel 20. The display panel 30 configured to display an ultrasound image and a GUI necessary for operations, is fixed on the front of the main body 10. The size of the portable ultrasound imaging apparatus 100 is not particularly limited, but the widest surface is supposed to be in a size of around A4 to B4, allowing an operator to place the apparatus on one arm and hold it by one hand.

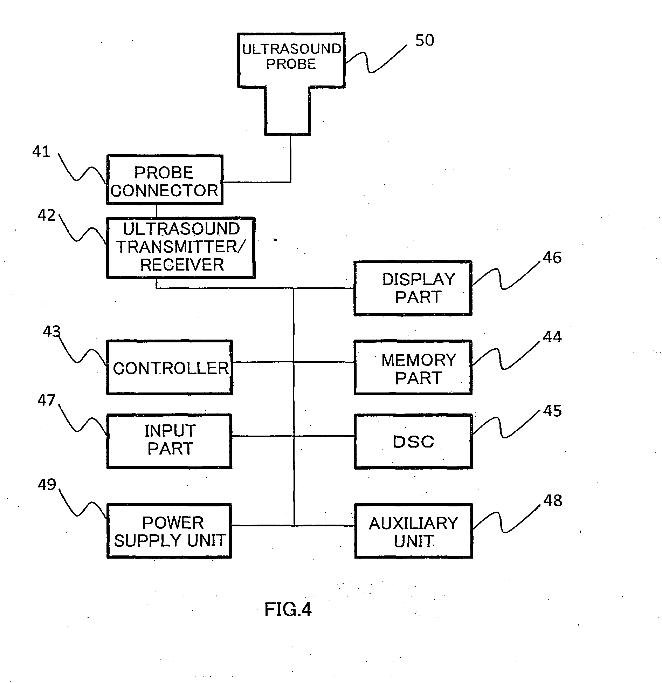

FIG. 4 illustrates an overview of the ultrasound imager 40 made up of the electronic circuit, and the like, being placed in the main body 10. The imager 40 incorporates an ultrasound probe (probe) 50, a probe connector 41 to which the probe 50 is connected, an ultrasound transmitter and receiver 42, a controller 43, a memory part 44, a DSC (digital scan converter) 45, a display part 46, an input part 47, an auxiliary unit 48, a power supply unit 49, and the like.

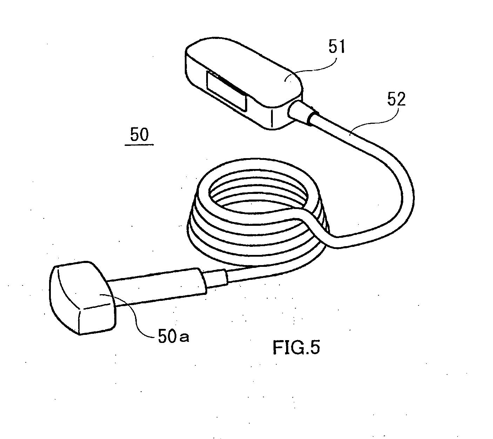

The probe 50 encloses within a case, an acoustic lens, an array in which piezoelectric elements for converting electric signals into ultrasound waves and also converts the ultrasound waves reflected from a subject into electric signals are arranged one-dimensionally or two-dimensionally, a backing material, and the like. There are prepared various types of probes appropriate for an examination target and depending on the situation. FIG. 5 illustrates one example of the probe 50. The probe 50 is made up of a probe main body 50a with a resin cover that covers the probe array, and the like, a connector 51 configured to connect the probe main body 50a to a power source, and a cable 52 connecting the probe main body 50a with the connector 51. When an ultrasound examination is performed, the connector 51 of the probe selected from the various types, is connected to the probe connector 41 provided in the main body 10, and the probe is used.

The ultrasound transmitter and receiver 42 transmits electrical signals to the probe 50 and receives the electrical signals from the probe 50, so as to perform processing such as phasing. The DSC 45 scans the received signals on the time axis, and performs a processing to display the received signals in the form of a two-dimensional image on the display panel 30. The display part 46 displays an ultrasound image and a GUI necessary for operations, and includes the display panel 30 and a driving unit thereof. The input part 47 is used to input instructions necessary for operating the apparatus, and buttons provided on the operation panel 20, and the like, are assigned those functions. The power supply unit 49 is provided with a terminal configured to supply power from a power source such as a battery, or from an external power source. The controller 43 controls the operations of each of the aforementioned parts. The memory part 44 stores data necessary for the operations of each part, and image data, and the like, in the middle of processing or as a result of the processing.

It is to be noted that the configuration of the imager 40 as shown in FIG. 4 is just an example, and other configurations may be applicable, like a configuration eliminating a part of the constitutional elements, or a configuration adding a different constitutional element.

With reference to the drawings from FIG. 1 to FIG. 3 again, an explanation will be provided as to appearance characteristics of the portable ultrasound imaging apparatus of the present embodiment.

As illustrated in FIG. 1, the front face of the main body 10 is made up of a region where the display panel 30 is fixed, and a region where the operation panel 20 is placed. As shown in FIG. 3, there are formed a level difference between the region where the display panel 30 is fixed, and the region where the operation panel is placed. In other words, as for the thickness of the main body 10, the thickness of the region where the display panel 30 is formed is thicker than that of the region where the display panel 30 is not formed. Space (stepped portion) 10a for placing the operation panel 20 therein is provided, next to the region where the display panel 30 is provided.

The operation panel 20 has substantially the same volume as the space of the stepped portion 10a, and it is mounted in a movable manner with respect to the main body 10. In the state where the operation panel 20 fits in the stepped portion 10a, the side surface of the operation panel 20 abuts against the stepped portion of the display panel 30, the front face of the main body 10 forms an almost continuous surface with the top surface of the operation panel 20, and three side surfaces of the operation panel 20 are almost continuous respectively to the corresponding side surfaces of the main body 10. With this configuration, the main body 10 and the operation panel 20 form an integrated tablet shape. The operation panel 20 is made to shift as appropriate from the stored position as shown in FIG. 1, and the angle with respect to the main body 10 is variable. A structure for mounting the operation panel 20 on the main body 10 will be described in detail later.

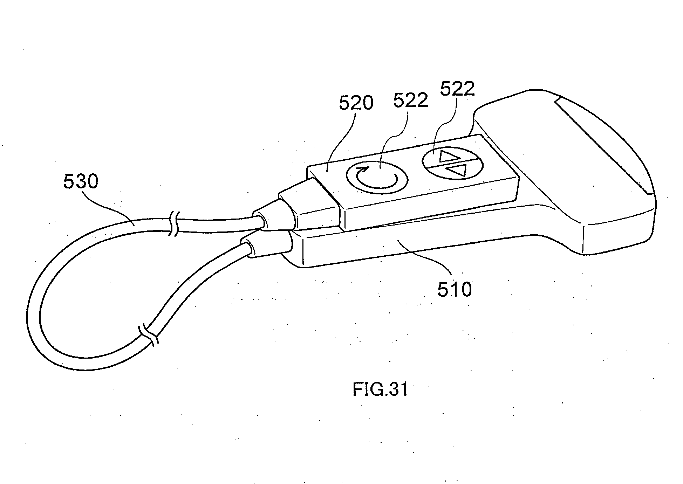

The operation panel 20 may be a touch-screen type, which converts the operations by an operator, such as pressing the button by a finger or a pen and dragging without letting up the button, into electrical signals, and transfers the signals to the controller 43 of the ultrasound imager 40. Since the portable ultrasound imaging apparatus 100 of the present embodiment is a tablet, the area thereof is limited, and thus there are provided operation buttons 21 to 27 (FIG. 31), the number of which is less than those of the operation panel in a conventional notebook ultrasound imaging apparatus. In the example as illustrated, there are provided seven operation buttons 21 to 27, and those operation buttons 21 to 27 implement plural functions by switching.

In order to complement the functions of the operation buttons mounted on the operation panel 20, the portable ultrasound imaging apparatus 100 of the present embodiment is provided with the operation buttons 81 and 82 on predetermined positions other than the operation panel, for example, on the handle and on the back surface. The operation button on the back surface is provided at the position that enhances the ease of operations in the case where the operator manages the apparatus using the handle 60 which will be explained later. Details of the operation button provided on the handle or on the back surface of the main body will be described below.

As illustrated in FIG. 2 and FIG. 3, there are mounted on the back side of the main body 10, the handle 60 that allows the operator to easily hold the main body 20, and the stand portion 70 configured to use the apparatus by placing the main body 10 on a desk or a table. The handle 60 and the stand portion 70 are mounted on the main body 10, each being foldable and rotatable, via the mounting mechanism placed substantially in the center of the back surface of the main body. On the back surface of the main body 10, there is formed a concave portion 15a to store the handle 60 and the stand portion 70 when they are folded. The depth of the concave portion 15a has substantially the same size as the thickness of the handle 60 and the stand portion 70, and when the handle 60 and the stand portion 70 are folded and stored in the concave portion 15a, they fit into the back surface. FIG. 2 and FIG. 3 illustrate the state that the handle 60 is stored in the concave portion 15a, and the stand portion 70 is pulled out of the storage 15a.

The operator holds the handle 60 by one hand, when the handle 60 is open with respect to the main body 10, allowing the operator to perform the ultrasound examination, while manipulating the operation panel 20 by the other hand. In this case, since the handle 60 is mounted rotatably with respect to the main body 10, the handle 60 may be placed at any position in the rotating direction. In addition, the handle 60 is usable not only when the measurement is performed, but also when carrying the portable ultrasound imaging apparatus 100.

When the stand portion 70 is opened with respect to the main body 10, this allows the main body 10 to be placed firmly on a table or on a desk. In this case, the stand portion 70 is rotated with respect to the main body 10, thereby enabling any of the placement of the main body 10, portrait and landscape.

On the back surface of the main body 10, there is further provided a port 17 for connecting the connector 51 of the probe (ultrasound probe) 50. In order to form the port 17 (the probe connector 41) on the back surface of the main body 10, each of both side surfaces of the main body 10 has a shape that a part of the back surface is recessed, and the port 17 is provided facing to this recessed space 15b, and further the space 15b serves as the space (storage) for placing the connector 51 therein. The thickness of the space 15b is substantially the same as the thickness of the connector 51. With this configuration, while the connector 51 is kept inserted into the port 17, the connector 51 is hidden in the back side when viewed from the front side of the main body 10, preventing protrusion of the connector from the side surface and keeping the connector from contact with an object or a person.

Considering the overview of the aforementioned ultrasound imaging apparatus of the present embodiment, each of the features provided in this ultrasound imaging apparatus will be described in detail. A key feature of the ultrasound imaging apparatus of the present embodiment includes that the handle and the operating portion are provided on the back surface of the apparatus main body, the operating portion being operable by the hand that holds the handle. In addition, the apparatus is characterized in the structure to mount the operation panel 20 on the main body 10, the mechanism for supporting the handle and the stand portion, the multi-functional operation buttons, the structure of the storage for the connector 51, and the like. Hereinafter, with reference to the drawings, the embodiments having those characteristics respectively will be explained.

### <First embodiment>

The portable ultrasound imaging apparatus of the present embodiment is characterized as having the operating portion (operation button) provided on the back surface. A conventional ultrasound imaging apparatus, including a notebook apparatus, is designed assuming that the apparatus is placed on a table, and therefore, all the operation buttons are supposed to be within the operation panel. The portable ultrasound imaging apparatus of the present embodiment has the operation button that is provided on the back surface or provided on the handle being mounted on the back surface, separated from the operation panel provided on the front surface.

The portable ultrasound imaging apparatus of the present embodiment may further be provided with the following characteristics: The operating portion includes the operation button that instructs to start and terminate the operation, or finalize the process. The operating portion includes the operation button having a trackball function. The operating portion is placed in the concave portion that is provided on the handle. Alternatively, it is placed in the concave portion that is provided on the back surface.

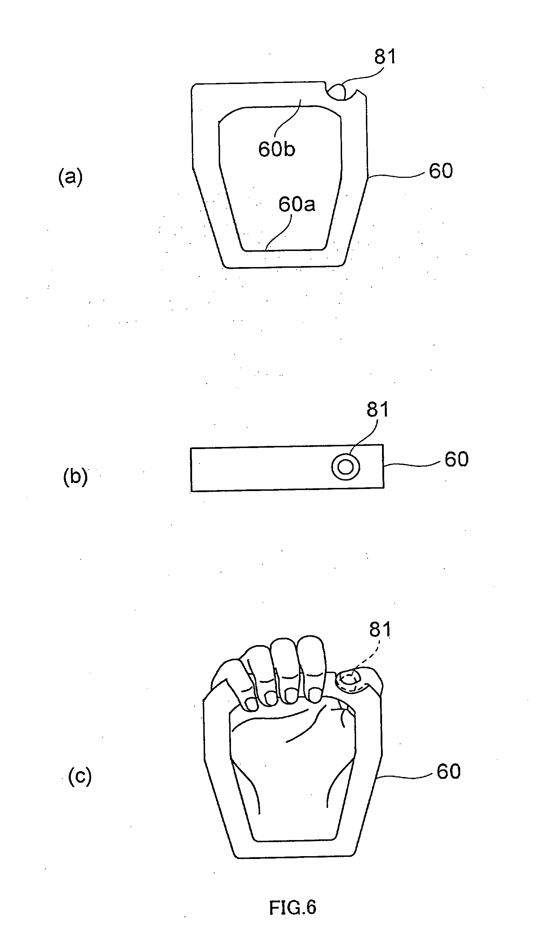

Hereinafter, with reference to FIG. 6 to FIG. 8, the present embodiment will be described in detail.

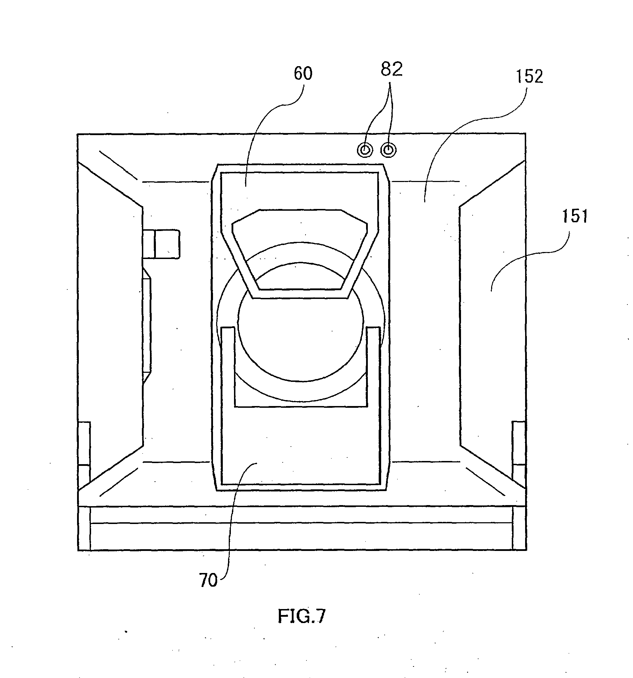

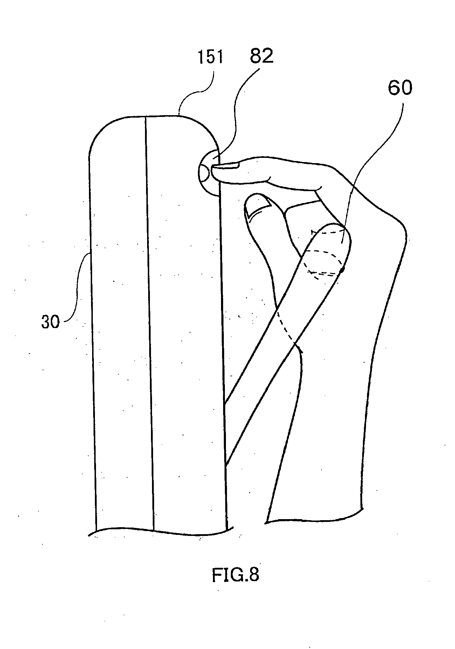

FIG. 6 illustrates an example that the operation button 81 is provided on the handle 60 itself. FIG. 6(a) is a plan view of the handle 60, FIG. 6(b) is a top view of a holder portion 60b of the handle, and FIG. 6(c) illustrates a usage state. FIG. 7 illustrates an example that the operation button 82 is provided on the upper end portion of the back side cover 152, and FIG. 8 illustrates the usage state thereof.

In the embodiment being illustrated, the handle 60 is mounted rotatably on the mounting mechanism on the back of the main body, the handle being made up of a mount 60a attached to the mounting mechanism and a holder 60b, and it has a circular shape as a whole. The operation button 81 is placed in the portion (concave potion) dented from the outer surface, on the edge of the holder 60b of the handle 60 having the aforementioned shape. The circular shaped handle 60 stores inside a cable (not illustrated) for establishing electrical connection between the operation button 81 and an electronic component (the imager 40) incorporated in the main body 10, whereby the operation button 81 is connected to the imager 40 via this cable and serves as the input part 47 (FIG. 4) of the imager 40.

In the embodiment in which the operation button 81 is provided on the handle 60, as shown in FIG. 6(b), the operator is allowed to manipulate the operation button 81 with his or her thumb, with grasping the handle 60 by the four fingers of the right hand, for instance. Since the operation button 81 is placed at the portion dented on the surface of the holder, there is no danger of malfunction while grasping the handle 60.

The operation button 82 that is provided on the back side cover is also electrically connected to the electronic component being the imager 40 via the cable within the back side cover, and the operation button serves as the input part 47 of the imager 40. Also in this example, it is preferable that the operation button 82 is placed in the concave portion formed on the back side cover. As illustrated in FIG. 8, the operator is allowed to manipulate the operation button 82 with his or her forefinger, for instance, while grasping the handle 60 with the thumb and the remaining fingers. FIG. 7 and FIG. 8 illustrate two operation buttons, but the number of the operation buttons may be one or more than two. It is desirable that the position and the number of the operation buttons are operable by the hand that holds the handle.

Either of the operation button 81 on the handle 60 and the operation buttons 82 on the back side cover 152 may be provided, or both may be provided.

The configurations of the operation buttons 81 and 82 are not particularly limited, but preferably, they are operated in a mechanical structure that converts a push-down operation into an electrical signal. The functions of the operation button are similar to the functions of the operation button A that is provided on the operation panel 20, such as starting/stopping the examination, freezing, and finalizing the processing operation, and further it is desirable that the functions are frequently used during the measurement. It is further possible to provide an operation button that has a function of trackball. As the operation button having the trackball operation, a ball-like protrusion provided in the concave portion is made rotatable, and a publicly known mechanism is employed for optically sensing the rotating direction and the rotating speed, thereby operating a cursor, or the like, displayed on the display part 30, in response to the rotating speed. The operation button 81 (82) having the trackball function allows operations by a single hand, to select a specific item from a menu displayed on the display part 30, or set an ROI on a specifically designated position on the displayed image. As an example, the operation button 81 placed on the handle 60 may be provided with the trackball function, and the operation button 82 placed on the back surface may be provided with a function for finalizing the processing.

As one usage pattern of the portable ultrasound imaging apparatus of the present embodiment, it is conceivable to hold the handle by one hand, placing the ultrasound imaging apparatus on the arm thereof, and to perform examination while treating the probe by the other hand. In that case, since the operation button having the aforementioned function is provided on the position that allows manipulation by the hand holding the handle, it is possible to perform processing such as suspending measurement, freezing, and recording, without using the hand holding the probe, i.e., without interrupting the examination using the probe, and this may drastically enhance the convenience of operation.

FIG. 7 illustrates the portable ultrasound imaging apparatus provided with the handle 60 and the stand portion 70 on the back side. A feature of the present embodiment is that the operating portion (operation button) is provided on the back side, and thus it is possible to eliminate the stand portion 70. The front side of the portable ultrasound imaging apparatus of the present embodiment is only required to have the display panel and the operation panel, and both the display panel and the operation panel may be stationary types, each being fixed on the main body, or any one of them may be a movable type.

In the portable ultrasound imaging apparatus of the present embodiment, separated from the operation panel on the front side of the main body, the operating portion is provided on the back side. Accordingly, when the handle of the apparatus main body is held by one hand and an ultrasound examination is performed while manipulating the probe by the other hand, the hand holding the handle is allowed to perform the operations easily. Therefore, it is not necessary to put the probe on a table or on a probe holder, in order to reserve the hand to manipulate the operation panel, facilitating the operations during the examination, and preventing interruption of the examination.

Furthermore, according to the portable ultrasound imaging apparatus of the present embodiment, the operating portion is placed in the concave portion provided on the handle or on the back surface, thereby preventing the malfunction, and guaranteeing the reliability of the operation.

Next, a structure other than the operating portion will be explained, which may be provided in the portable ultrasound imaging apparatus of the present embodiment.

### <Mounting structure of the operation panel 20>

The portable ultrasound imaging apparatus of the present embodiment is provided with the operation panel 20 on the same plane as the display panel 30, separated from the operating portion on the back side of the main body. The operation panel 20 is slidable (movable within the same plane) with respect to the main body 10, and it is rotatable with respect to the main body 10, at any point in the sliding direction. Specifically, the main body is provided with the stepped portion that allows the operation panel to be stored along the surface where the screen of the display panel is formed, and also provided with a support mechanism configured to support the operation panel in a slidable manner in the direction parallel to the screen of the display panel, and further support the operation panel in such a manner as varying the angle with respect to the screen. The shape of the stepped portion is almost equivalent to the shape of the operation panel, and under the condition that the operation panel is stored in the stepped portion, the screen and the operating surface of the operation panel are substantially in the same plane.

The support mechanism of the operation panel is provided with a slide mechanism and a tilt mechanism, and the operation panel is fixed on the tilt mechanism, and the tilt mechanism is rotatably coupled with the slide mechanism.

As a way of example, the slide mechanism includes a plate-like member on which a long hole is formed for sliding in the longitudinal direction, and a guide pin that is fixed on the main body and engaged with the long hole for guiding the plate-like member to move. The tilt mechanism is provided with a rotating member that is rotatably supported on the plate-like member by the axis being orthogonal to the slide direction and parallel to the screen, and the operation panel is fixed on the rotating member.

The stepped portion of the main body for storing the operation panel may have a panel part being parallel to the screen, and in that case, a slit is formed on the panel part, allowing the plate-like member to move in the slide direction. The portion where the long hole of the plate-like member is formed is engaged with the guide pin on the side being opposite to the screen of the panel part, and it is possible to configure such as supporting the rotating member on the screen side.

The slide mechanism and the tilt mechanism may be connected via a link. In this case, the operation panel may be movable from the position where it is stored in the stepped portion to the position that faces to the back side of the main body.

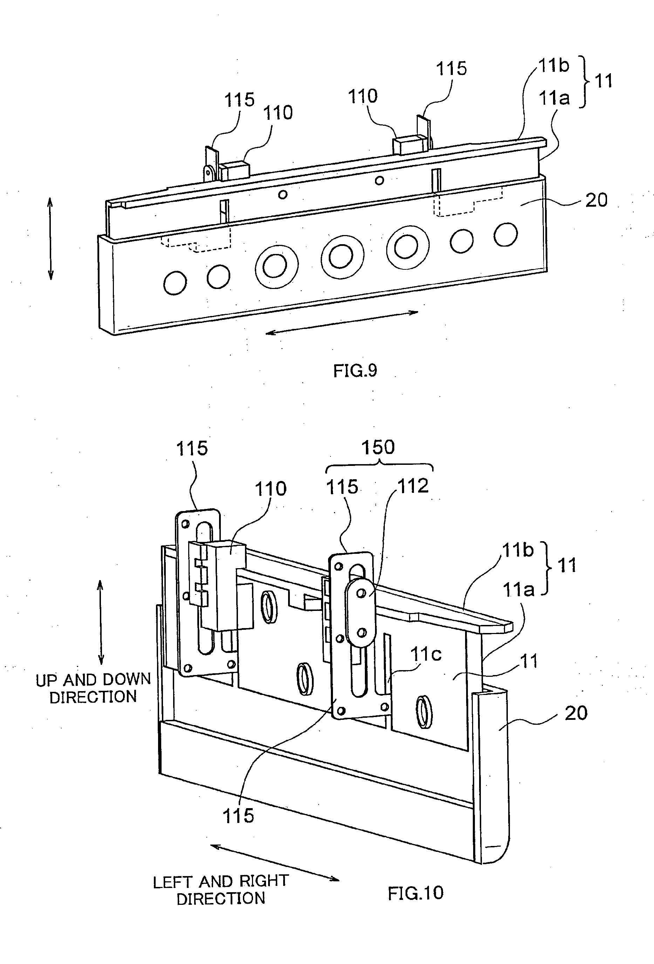

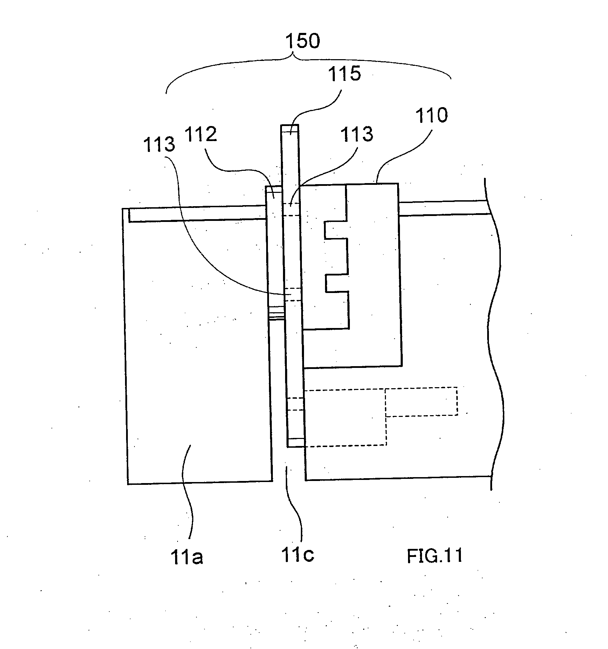

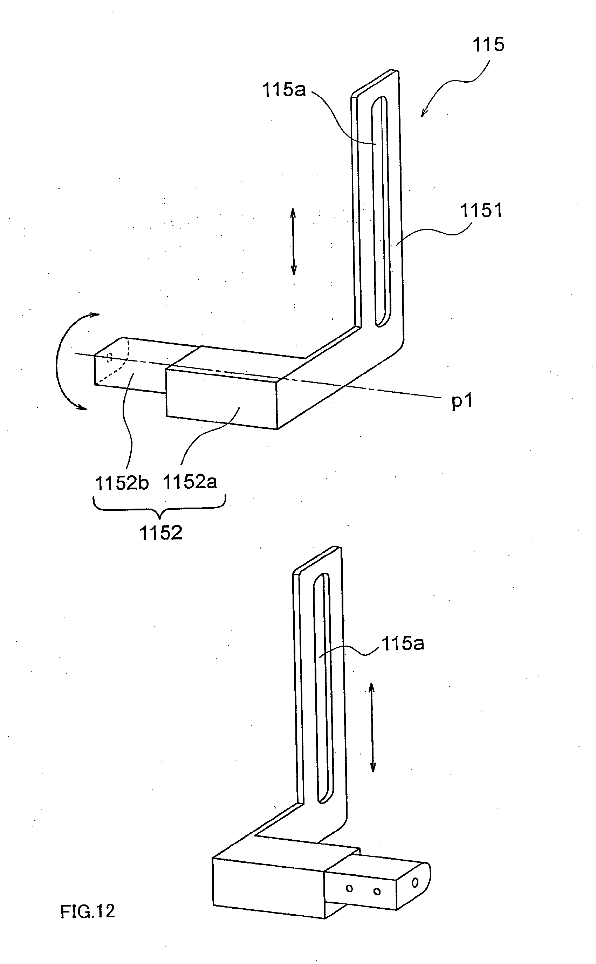

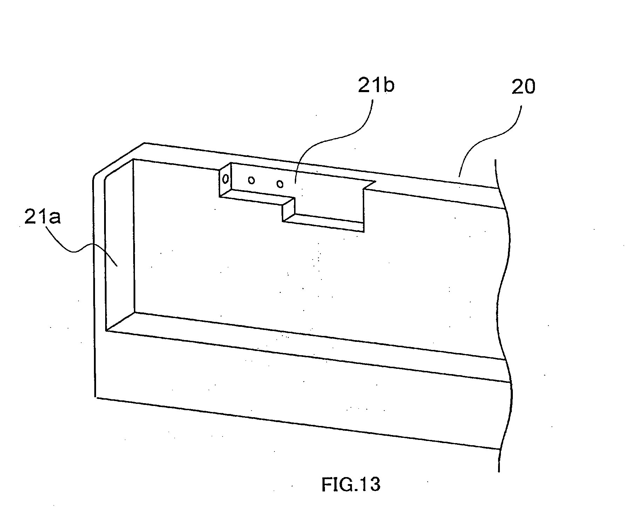

Hereinafter, with reference to the drawings, the portable ultrasound imaging apparatus of the present embodiment will be further explained. FIG. 9 to FIG. 13 illustrate an example of the mounting structure that enables the movement of the operation panel 20 described above. FIG. 9 and FIG. 10 are a perspective view from the front side and a perspective view from the back side, respectively, of a part of the front face of the main body 10, FIG. 11 and FIG. 12 illustrate the essential portion of the mounting mechanism, FIG. 11 is a front view that is viewed from the inside, and FIG. 12 is a perspective view of the mounting member, viewed from the front side and the back side. FIG. 13 illustrates a back side structure of the operation panel. In FIG. 9 and FIG. 10, the up-down direction (or the vertical direction) and the right-left direction (or the horizontal direction) are indicated by the arrows. Those are just provisional directions for explanation, and they may be changed to any direction according to the posture of the portable ultrasound imaging apparatus of the present embodiment. The names of the vertical part and the horizontal part may also be variable.

A front panel 11 for providing the region 10a to store the operation panel 20 is fixed on the front surface of the main body 10, and the mounting structure of the operation panel 20 is fixed on the front panel 11.

The front panel 11 of the main body 10 may be a plate member made of rigid metal, FRP, and the like, made up of the plate member 11a having the plane parallel to the main-body front face (referred to as the vertical part), and the horizontal part 11b integrally mounted on the edge thereof (the upper end thereof in the figure). The vertical part 11a is fixed on the structure of the main body 10, via a screw, or the like. On the vertical part 11a, there are formed one or plural holes for passing a cable therethrough (not illustrated) to establish electrical connection between the operation panel 20 and the imager (electronic circuit) 40 that is stored in the main body 10.

The display panel 30 is placed on the horizontal part 11b of the front panel 11, and a pair of blocks 110 being the mounting structure of the operation panel 20, are fixed on the back side. In the illustrated embodiment, the block 110 has the shape of rectangular parallelepiped, a part of which fits into the rectangular-shape notch formed on the horizontal part 11b of the front panel, and fixed on the vertical part 11a via the screw, or the like. As illustrated in FIG. 11, a pair of pins 113 are fixed on each of the blocks 110. There is formed a long hole on the slide member 115 on which the operation panel 20 is fixed, and the pair of pins 113 are mounted on the block 110 via a stopper 112, in such a manner that those pins pass through the long hole. The pins 113 serve as the guide for moving the slide member 115 in the vertical direction. The stopper 112 adjusts the clamping force of the slide member 115 against the block 110, thereby imposing predetermined resistance to the movement of the slide member 115 in the slide direction (the vertical direction), and this allows the slide member 115 to stop at any position in the slide direction.

The slit 11c is formed on the vertical part 11a of the front panel 11, allowing the slide member 115 to move in the vertical direction. The slide members 115 are provided respectively on the right and left blocks 110. Since the structure of each of the blocks 110 is identical, only one slide member 115 will be explained in the following.

As illustrated in FIG. 12, the slide member 115 has an L-shaped plate 1151 on which the long hole 115a is formed, and a shaft 1152 being bent by 90 degrees with respect to the surface of the plate 1151, and a pair of pins 113 pass through the long hole 115a of the plate 1151, thereby allowing the slide member 115 to slide in the vertical direction without rotating.

The shaft 1152 of the slide member 115 is made up of the fixed part 1152a and the rotor 1152b being a hinge structure. The dimension of the fixed part 1152a is larger than the rotor 1152b in the vertical direction, and fixed part is formed integrally with the plate 115a. The rotor 1152b is rotatably mounted on the fixed part 1152a about the axis p1 (not illustrated) provided inside the shaft 1152. The upper end of the operation panel 20 is fixed on the rotor 1152b. A torque hinge may be employed as the shaft 1152 having such structure, which is able to stop the rotation at any position, and a torque value of the torque hinge may be selected appropriately, considering the weight of the operation panel 20.

FIG. 13 illustrates a back side structure of the operation panel 20 with which the slide member 115 is coupled. As illustrated, if the side where the operation buttons (21 to 27 in FIG. 31) of the operation panel 20 are provided is assumed as the front face, a flat part 21a that has a level difference relative to the outermost surface of the back side, is formed for placing the vertical part 11a of the front panel 11. On the upper end of the flat part 21a, there is formed a concave port 21b having the shape corresponding to the shape of the shaft 1152 (the fixed part 1152a and the rotor 1152b), and the shaft 1152 projecting forward from the vertical part 11a of the front panel 11 is placed within the concave part 21b. The rotor 1152b of the shaft 1152 is fixed on the position corresponding to the concave part 21b via the screw, or the like. Since the size of the fixed part 1152a is made different from the size of the rotor 1152b, displacement in the lateral direction is prevented, and further the fixed part 1152a is held by a surface, thereby preventing a turn and maintaining the posture.

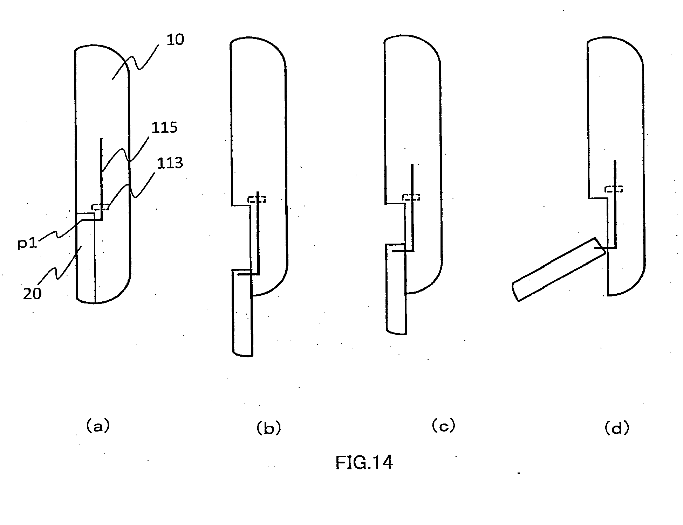

With reference to FIG. 14, an explanation will be provided as to the movement of the operation panel 20 mounted on the main body 10 according to the mounting structure as described above. As illustrated in FIG. 14(a), in the state (at the initial position) that the operating surface of the operation panel 20 is parallel to the front surface of the main body 10 and the operation panel 20 substantially abuts the display panel 30, when the operation panel 20 is drawn with holding both edges, downwardly in the figure, the slide member 115 that is fixed to the operation panel 20 moves integrally with the operation panel 20. This movement goes along the direction in which the long hole 115a of the slide member 115 is guided by the pin 113s, in other words, the vertical direction. Since the slide member 115 (the plate 1151) is fixed with a predetermined clamping force, the movement may be stopped at any position. FIG. 14(b) illustrates the state that the operation panel 20 has moved to the lowermost end.

When the lower side of the operation panel 20 is pulled out of the main body 10, for instance, at a desired position of the operation panel 20 in the vertical direction (FIG. 14(c)), the rotor 1152b of the shaft 1152 on which the operation panel 20 is fixed rotates with respect to the other parts (the plate 1151 and the fixed part 1152a of the shaft 1152) of the slide member 115, thereby tilting the operation panel 20 relative to the main body 10. This may establish the tilted state as illustrated in FIG. 14(d).

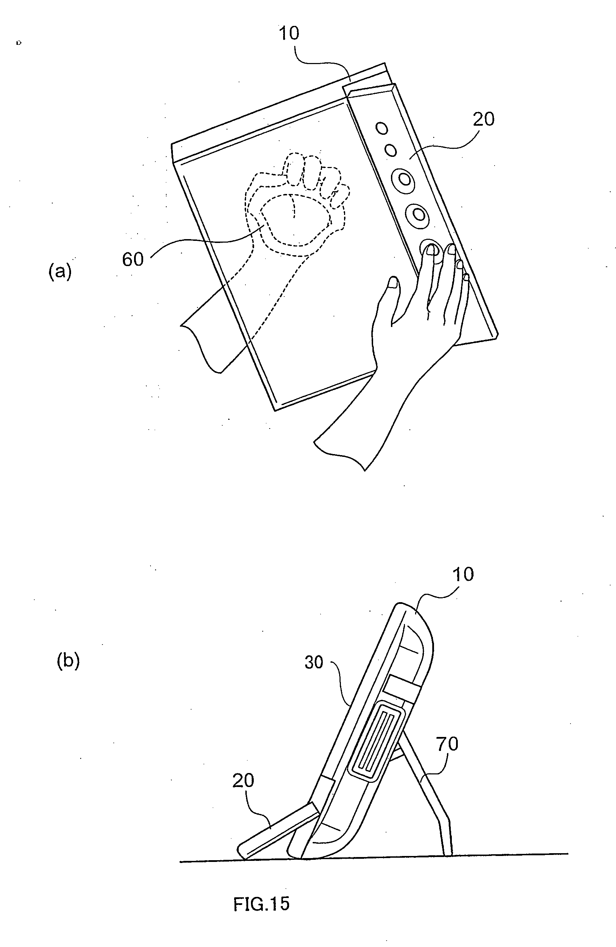

As described above, since the operation panel 20 is made slide and tilt at any position relative to the main body 10, the operator is allowed to move and operate the operation panel 20 at a position facilitating the operation, depending on the usage state. By way of example, as shown in FIG. 15(a), in order to allow the operator to hold the handle 60 on the back side of the main body 10 and use the apparatus being placed on the arm, the operation panel 20 is tilted as appropriate for using the apparatus, in response to the angle of the arm with respect to the body of the operator, thereby enhancing the operability.

Alternatively, as illustrated in FIG. 15(b), when the apparatus is treated on the table, while setting up the stand portion 70 on the back side of the main body 10, the operation panel 20 is made slide and tilt in response to the angle of the display panel 30 (screen), the angle being given by the stand portion 70, thereby causing the lowermost end of the operation panel 20 to abut against the table, allowing the apparatus to stably stand on the table along with the stand portion 70, and further enhancing the operability of the operation panel. In this case, even when the angle varies with respect to the surface on which the main body 10 is placed, the slide position and the tilt angle may be adjusted, thereby causing the edge of the operation panel 20 to abut against the placement surface, and this allows the operation panel 20 and the stand portion 70 to maintain a stable posture of the main body, ensuring stable operation of the operation panel 20.

As described so far, according to the mounting structure of the operation panel 20 of the present embodiment, the operation panel is allowed to slide and tilt with respect to the main body 10, and further allowed to tilt at any position in the slide direction, thereby enhancing the operability of the portable ultrasound imaging apparatus.

The moving range of the operation panel 20 in the slide direction assumes as the upper limit, the position where the upper end of the operation panel 20 abuts against the horizontal part 11a of the front panel 11 extending forward. The lower limit may be restricted by the length of the long hole (from the level where the operation panel 20 is fixed, to the upper end of the long hole 115a). In the mounting structure of the present embodiment, the lower limit is restricted to the position where the upper end of the operation panel 20a is a little higher than the lower end of the front panel 11, but the length of the slide member 115 and the long hole 115a formed thereon may be extended upwardly, thereby expanding the movable range on the lower limit side.

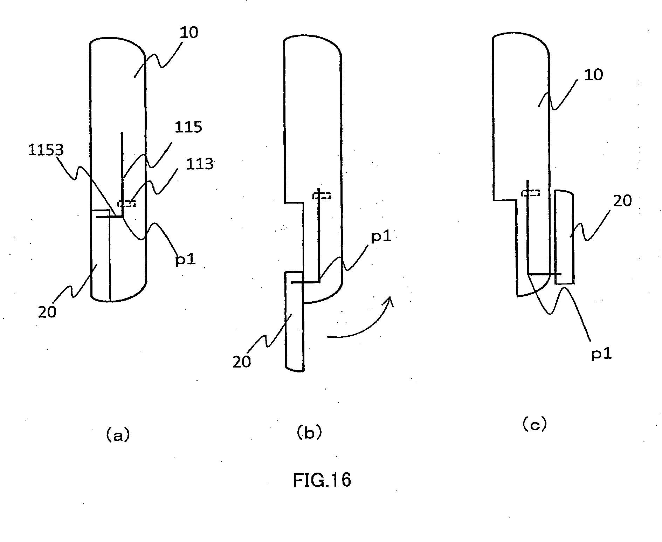

In the present embodiment, the rotation axis p1 of the operation panel 20 is configured as being positioned on the front side of the main body with respect to the plate 1151. However, as illustrated in FIG. 16(a), it is further possible to employ the configuration that the rotation axis p1 of the operation panel 20 is positioned on the extended line of the long hole 115a of the plate 1151 in the longitudinal direction, and coupling the rotation axis p1 and the operation panel 20 via the link 1153. In this case, when the rotation axis of the operation panel 20 is made slide down to the lower end of the front panel 11 (FIG. 16(b)), the operation panel 20 is turned approximately by 180 degrees about the rotation axis p1, thereby enabling the operation panel to be positioned on the back side of the main body 10 (FIG. 16(c)). In this posture, for example, along with showing a patient, the image displayed on the display panel 30 on the front side of the main body 10, the operator is allowed to manipulate the operation panel 20 from the back side, so as to change the display (e.g., expand the image and/or displace the image, etc.).

The mounting structure as shown in the drawings from FIG. 9 to FIG. 13, is one example of the mechanism that slides and tilts the operation panel 20. As far as the structure is provided with a mechanism to slide the operation panel 20 with respect to the main body 10, and a rotating mechanism incorporated in the slide mechanism, the structure is not limited to those as illustrated. By way of example, it is further possible to employ a structure that a guide rail is provided in the vertical direction on the front panel 11, preparing a mounting member made up of a slider moving up and down along the guide rail fitting thereto and a rotor mounted on the slider in a rotatable manner, and to fix the upper end of the operation panel 20 to the rotor of this mounting member.

Since most of the mechanism as shown in the figures from FIG. 9 to FIG. 13 is provided on the back side of the front panel 11, there is no danger of pinching a finger, a cord, or the like, in the guide rail, or the like, and there is also an advantage that a compact mechanism is able to be established.

### <Mounting structure of the handle 60 and the stand portion 70>

The portable ultrasound imaging apparatus of the present embodiment is characterized in that the back surface of the apparatus main body is provided with a handle and/or a stand portion forming variable angle with the back surface, and being rotatable about the axis that is perpendicular to the back surface of the main body 10.

When the portable ultrasound imaging apparatus of the present embodiment is manipulated while being placed on the arm, the handle 60 held by the hand stabilizes the usage posture, and further the handle is usable for carrying the ultrasound imaging apparatus. When the portable ultrasound imaging apparatus of the present invention is used while being placed on the table, the stand portion 70 supports the apparatus, and stabilizes the usage posture at any angle with respect to the table surface. It is possible to provide only either one of the handle 60 and the stand portion 70. If an appropriate shape is formed, both functions of the handle and the stand portion may be provided by one shape. In the following, an explanation will be provided as to an embodiment that is provided with both the handle 60 and the stand portion 70. In addition to the aforementioned characteristics, the portable ultrasound imaging apparatus of the present embodiment has a characteristic that the handle is rotatable being independent of the stand portion.

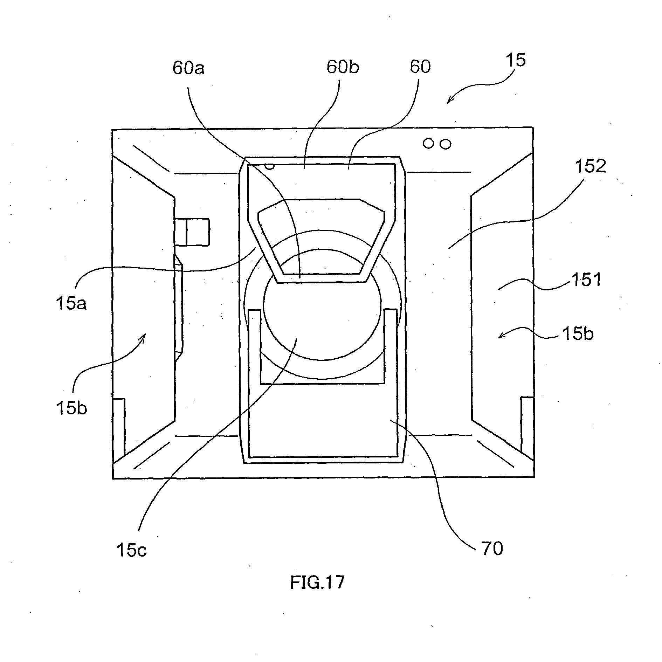

Hereinafter, with reference to the drawings from FIG. 17 to FIG. 23, structures of the handle 60 and the stand portion 70 of the portable ultrasound imaging apparatus will be explained. FIG. 17 is a rear view of the ultrasound imaging apparatus, and an explanation will be provided assuming temporarily that the four orientations; up, down, left, and right on the paper, respectively correspond to those four orientations of the apparatus in FIG. 17. However, the orientation of the apparatus may vary freely, and the orientations used for the explanation will not restrict those of the apparatus.

As illustrated in FIG. 17, the back side surface 15 of the portable ultrasound imaging apparatus of the present embodiment is provided with a back side panel 151 covering the back side of the display panel 30 and the back side of the front panel 11 continuing thereto, and a back side cover 152 covering the imager 40. The back side panel 151 is parallel to the screen of the display panel 30 and the front panel 11, and the horizontal size and the vertical size are substantially equal to the horizontal size and the vertical size of the apparatus. The back side cover 152 has a projecting shape (convex-like portion) to cover a part of the back side panel 151, and the upper end and the lower end are respectively connected to the upper end and the lower end of the back side panel 151. The left and right ends have the shape getting inward relative to the back side panel 151, except the portions on the four edges where curved surfaces (R) are formed. In other words, the left and right sides of the back side surface form concave portions 15b, formed by the back side panel 151 and the side surfaces of the back side cover 152.

The back side cover 152 having the concave portions 15b on both sides forms space configured to accept the connector 51 of the probe 50 which will be described later. Another embodiment is possible to provide a port for the connector 51 of the probe 50 on the side surface of the apparatus. In this case, the concave portion 15b is not absolutely necessary. Further in this embodiment, the concave portions 15b are provided respectively on the left and right sides of the apparatus, but the number of concave portions, the shape and the position of the concave portion may be changed appropriately.

On the center of the back side cover 152, there is provided a concave portion 15a having a rectangular shape whose four corners are curved. Further on the center of the concave portion 15a, there is provided a circular-shaped opening, and the mechanism of the handle 60 and the stand portion 70 is mounted within this opening. In FIG. 14, the opening is covered with the circular shaped cover 15c.

The handle 60 and the stand portion 70 are stored within the concave portion 15a in the folded state, i.e., under the condition that they are made flat relative to the back surface, and the ultrasound imaging apparatus is allowed to maintain the tablet-like shape, without any projections from the back surface. The handle 60 has a ring-like shape, and a part of the ring is a base 60a fixed on the apparatus, and another part (holder part) 60b is to be held by a person, which serves as a handle by pulling the holder part 60b out of the back surface. The stand portion 70 is also pulled out of the apparatus, and it functions as a stand as shown in FIG. 2.

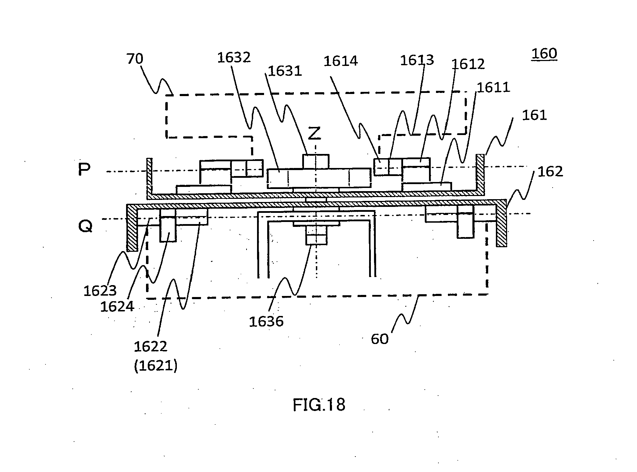

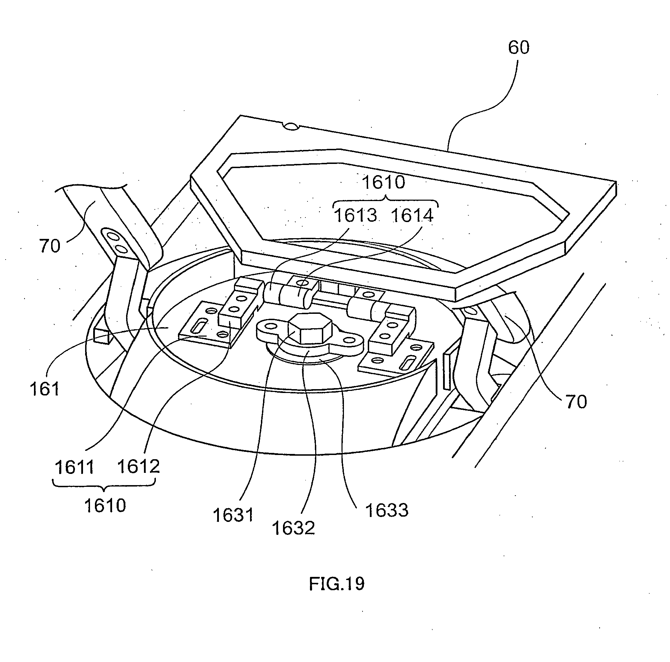

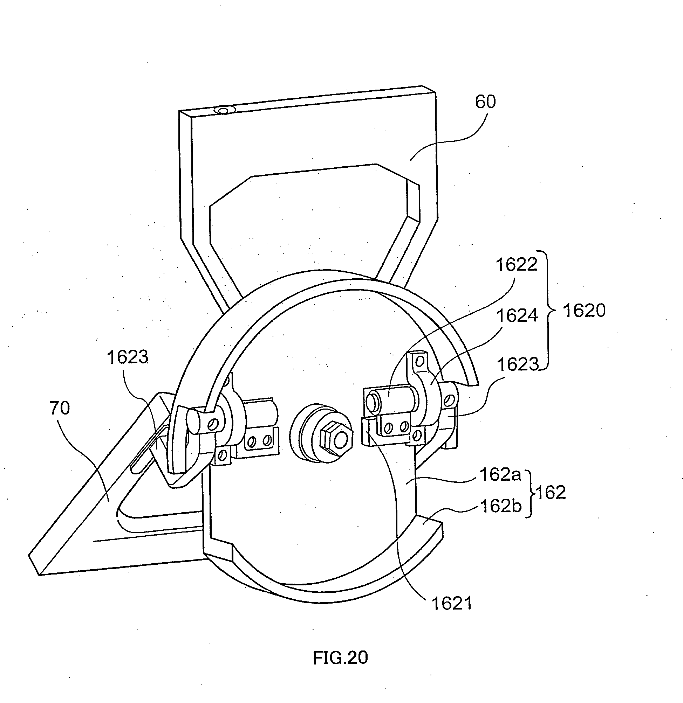

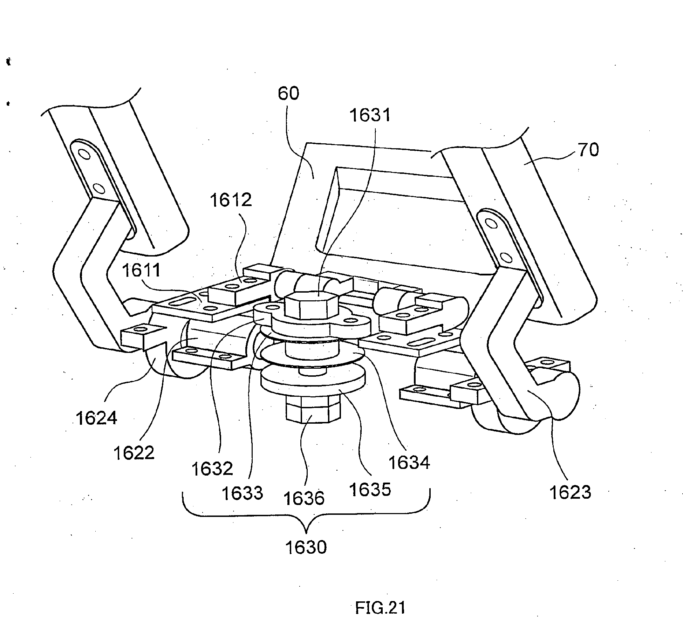

With reference to the drawings from FIG. 18 to FIG. 21, the mounting mechanisms of the handle 60 and the stand portion 70 enabling the operations above will be explained. FIG. 18 is a cross-sectional view of the mounting mechanisms of the handle and the stand portion, FIG. 19 is a perspective view showing the mounting mechanism 1610 of the handle, FIG. 20 is a perspective view showing the mounting mechanism 1620 of the stand portion, and FIG. 21 is a perspective view showing a rotation mechanism 1630 for mounting the handle and the stand portion. It is to be noted that in the cross-sectional view in FIG. 18, the handle 60 and the stand portion 70 are schematically represented by dotted lines only. In FIG. 19, the stand portion 70 is not fully illustrated.

As shown in FIG. 18, the mounting mechanisms of the handle and the stand portion are provided with a shaft Z (not illustrated) fixed on the structure of the main body 10 (or the back side panel 151), and the first rotary plate 161 and the second rotary plate 162, in a disc-like shape, each rotatably mounted with respect to the shaft Z. The handle 60 is fixed on the upper surface of the first rotary plate 161 via the mounting mechanism 1610 (1611 to 1614), and the stand portion 70 is fixed on the lower surface of the second rotary plate 162 via the mounting mechanism 1620. As shown in FIG. 17, those mounting mechanisms 1610 and 1620 are covered by the cover 15c being integral with the back side cover 152 from a viewpoint of design, and they are invisible in appearance.

As shown in FIG. 18 and FIG. 19, the mounting mechanism 1610 of the handle 60 is made up of a pair of plates 1611 fixed on the first rotary plate 161, a hinge (1612, 1614) fixed on each of the plates 1611, and a washer 1613. The hinge is made up of a shaft fixture 1612 for fixing a shaft (not illustrated), and a rotor 1614 being supported rotatably with respect to the shaft, the base 60a of the handle 60 being fixed on the rotor, and the washer 1613 is interposed between the shaft fixture 1612 and the rotor 1614. The rotors 1614 on the left and right are integrated being coupled via a plate member, and the base 60a of the handle 60 is fixed on the plate member of the rotors, via a screw, or the like. The shaft fixed on each shaft fixture 1612 exists on the same axis P that connects the shaft fixtures on both sides, and the handle 60 fixed on the rotors 1614 rotates about this axis P.

According to the mounting mechanism 1610, the handle 60 rotates about the axis P, with respect to the rotary plate 161, allowing the holder part 60b being opposed to the base 60a to move rotationally from the state of being stored within the concave portion 15a to the state of being raised outwardly from the concave portion 15a. Under this condition, the operator is allowed to grasp the holder 60b and manipulate the apparatus while placing the apparatus on the arm, or carry the apparatus. The washer 1613 restrains with its clamping force, the rotation of the handle 60 about the axis P, in other words, it imposes resistance to the rotational movement of the holder 60b, allowing the handle 60 to be used in the stationary state at any angle.

As illustrated in FIG. 20, the second rotary plate 162 is provided with a circular plate 162a positioned on the lower side of the first rotary plate 161, and a ring 162b on the periphery of the circular plate 162a. Arcs on two portions opposed to each other of the circular plate 162a are cut off to form linear parts, and the stand portion 70 is fixed via the mounting mechanism 1620 in such a manner as positioned on both sides of those linear parts. The portions of the ring 162b respectively associated with the linear parts of the circular plate 162a are notched, and this allows movement of the mounting parts of the stand portion 70, which will be described later.

As illustrated in FIG. 18 and FIG. 20, the mounting mechanism 1620 of the stand portion 70 is made up of a pair of fixed blocks 1621 being fixed on the back surface of the circular plate 162b, shaft fixtures 1622 respectively fixed on the fixed blocks 1621, a shaft (not illustrated) fixed on the shaft fixtures 1622 and positioned on the axis Q, arm parts 1623 rotatably connected to the shaft fixtures 1622 via the shaft, and supporters 1624 configured to impose resistance to the movement around the shaft of the arm parts 1623. The arm part 1623 has a hook-like shape, and one end is axially supported by the shaft of the shaft fixtures 1622, and the stand portion 70 is fixed on the other end.

As shown in FIG. 20, with this mounting mechanism, the stand portion 70 fixed on the arm parts 1623 is allowed to rotationally move from the state of being stored within the concave portion 15a to the state of being raised outwardly from the concave portion 15a. In addition, since the arm part 1623 has the hook-like shape, though the rotation axis Q of the stand portion 70 is located lower than the rotation axis P of the handle 60 with respect to the back surface, the handle and the stand portion are able to form a flat plane that substantially continues to the back surface, when they are stored in the concave portion 15a. In addition, when the stand portion 70 is rotated about the shaft Z by the rotation mechanism described later, while the stand portion 70 is tilted from the back surface, it is possible to avoid hampering the rotation by the interference between the stand portion 70 and the inner wall of the concave portion 15a on the back surface.

Next, the rotation mechanism 1630 including the first rotary plate 161 and the second rotary plate 162 will be explained. The first rotary plate 161 on which the handle 60 is fixed and the second rotary plate 162 on which the stand portion 70 is fixed are arranged in parallel to the back surface, inside the back surface 15 of the main body 10. As shown in the cross sectional view of FIG. 18, the shaft Z passes through each center thereof in the direction perpendicular to the back surface, and the first rotary plate and the second rotary plate are supported rotatably within the plane being parallel to the back surface, assuming the shaft Z as the rotation axis. As shown in FIG. 21, a nut 1631 is fixed on one end of the axis 160, and the other end is fixed on the structure of the main body 10, via a double nut 1636. The first rotary plate 161 is placed between the fixture 1632 for fixing the nut 1631 and the first washer 1633, and the second rotary plate 162 is placed between the first washer 1633 and the second washer 1634. Then, clamping force applied to each washer adjusts the frictional force (resistance) of the rotation about the shaft Z. In the present embodiment, the frictional force onto the second rotary plate 162 is adjusted to be larger than the frictional force onto the first rotary plate 161. In other words, the rotary torque of the first rotary plate is smaller than the rotary torque of the second rotary plate.

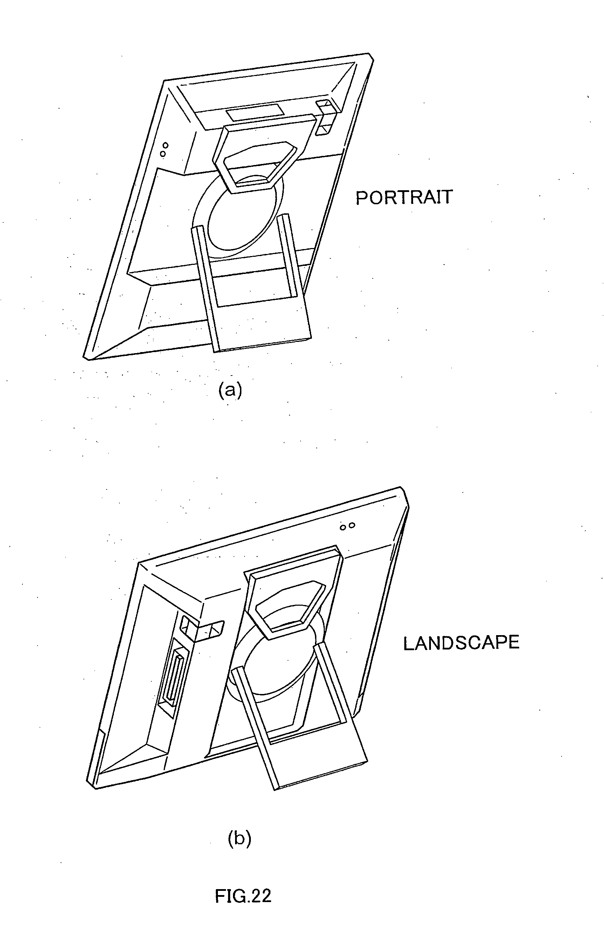

With this structure, under the condition that the stand portion 70 and the handle 60 are raised outwardly from the concave portion 15a, the stand portion 70 is manipulated rotationally, thereby allowing the second rotary plate 162 to rotate around the shaft Z. Accordingly, as shown in FIG. 22(a) and FIG. 22(b), it is possible to use the apparatus, placing the main body 10 on the table, in any of the following postures; in portrait mode or in landscape mode. When this stand portion 70 moves rotationally, the first rotary plate 161 is rotated integrally with the second rotary plate 162.

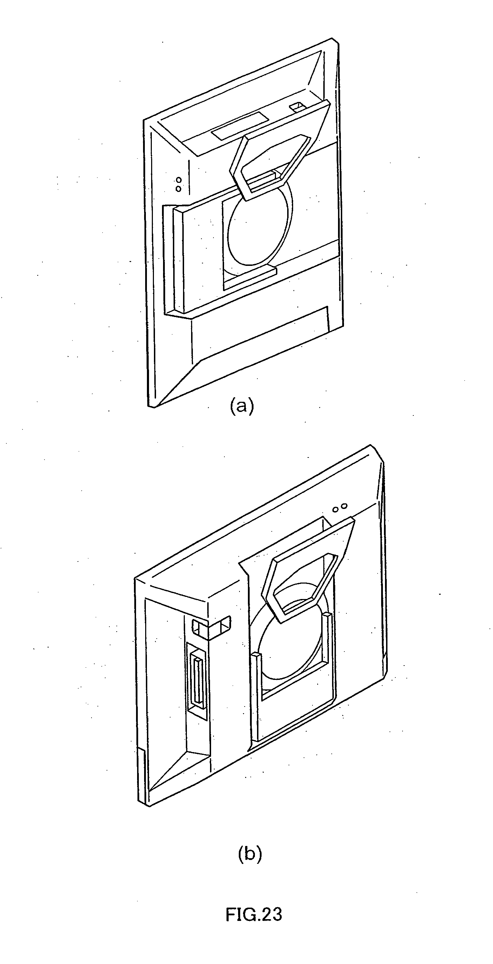

On the other hand, when only the handle 60 is raised up outwardly and manipulated rotationally, under the condition that the stand portion 70 is stored in the concave portion 15a, the second rotary plate 162 on which the stand portion 70 is fixed does not rotate, since the rotation of the stand portion 70 is restricted by the side surface of the concave portion 15a. Therefore, only the first rotary plate 162 and the handle 60 fixed thereon rotate, thereby allowing the handle 60 to move to any position in the rotating direction. With this configuration, as illustrated in FIG. 23(a) and FIG. 23(b), it is possible to hold the handle 60 in any of the states; portrait or landscape, and even when it is used while being placed on the arm, for instance, the angle with respect to the main body may be changed arbitrarily.

In the present embodiment, as described above, the frictional force on the rotation around the shaft Z of the first rotary plate 161 and the second rotary plate 162 is adjusted in such a manner as being larger on the second rotary plate 162 than the first rotary plate 161. Therefore, even in the state where the stand portion 70 is raised and rotatable, it is possible to rotate only the handle 60 independently.

As explained so far, the portable ultrasound imaging apparatus of the present embodiment is provided with the handle 60 and the stand portion 70 being able to be raised up from the back surface, on the back side of a tablet structure, and they are rotatable independently of each other, with respect to the main body 10. With this configuration, the apparatus 10 is usable in any posture; portrait or landscape, and further the angle between the handle 60 and the back surface when holding the handle 60, and the angle when the stand portion 70 is raised, may be set arbitrarily. Accordingly, this configuration may enhance the usability.

In the present embodiment, the frictional force of the rotation mechanism of the handle 60 is configured as different from the frictional force of the stand portion 70, against the main body 10. However, if they are rotatable independently of each other, such difference in the frictional force is not necessarily provided.

In the present embodiment, it is explained that the rotation axis of the handle 60 coincides with the rotation axis of the stand portion 70. However, it is further possible to configure such that they are supported by different axes with respect to the main body. In this case, either of the handle 60 and the stand portion 70 is folded and stored into the concave portion 15a, and the other one is raised up from the concave portion 15a and rotated at any angle for use, thereby preventing interference therebetween. However, the embodiment as illustrated in the drawings is preferable, in the points that the structure is compact and flexibility in usage is high.

### <Mounting structure of the probe connector>

Next, the mounting structure of the probe connector will be explained. The portable ultrasound imaging apparatus of the present embodiment is characterized in that the back surface is provided with space for placing therein a connector part of the probe, when the probe is connected. In other words, the main body is provided with a convex part with the maximum thickness equal to or larger than the thickness of the connector part and having a narrowed width portion that is narrower in width relative to the main body, on the second surface (back surface) on the other side of the first surface (front surface) on which the display panel is provided. In the narrowed width portion of the convex part, there is formed a probe connector to connect the connector part.

Alternatively, the main body is provided with a storage part that stores the connector, on the second surface (back surface) on the other side of the first surface (front surface) on which the display panel is provided. A probe connector to which the connector part is connected is formed on the surface (side surface) being orthogonal to the second surface that faces to the storage part.

FIG. 5 illustrates one example of the probe, but there are various types of probe, depending on an examination target and an examination purpose. However, the number of the terminals and the shape of the connector 51 are substantially in common, and an identical size is prepared, allowing any connector to share the port.

A conventional apparatus, such as a notebook ultrasound imaging apparatus, in which the display panel is openable and closable with respect to the apparatus main body, the port of the probe connector is provided on the side surface of the apparatus main body. When the probe is connected to the port, the connector is placed at a position protruding from the side surface of the main body. In this case, while at work, there is a possibility that the connector bumps against an external structure or a person, and this may render the connection unstable, or damage the connector. In the ultrasound imaging apparatus of the present embodiment, the back side panel 151 and the back side cover 152 form the space for storing the probe connector on the back side, and the port is provided facing to this space, thereby protecting the connector from the aforementioned collision with the structure or person.

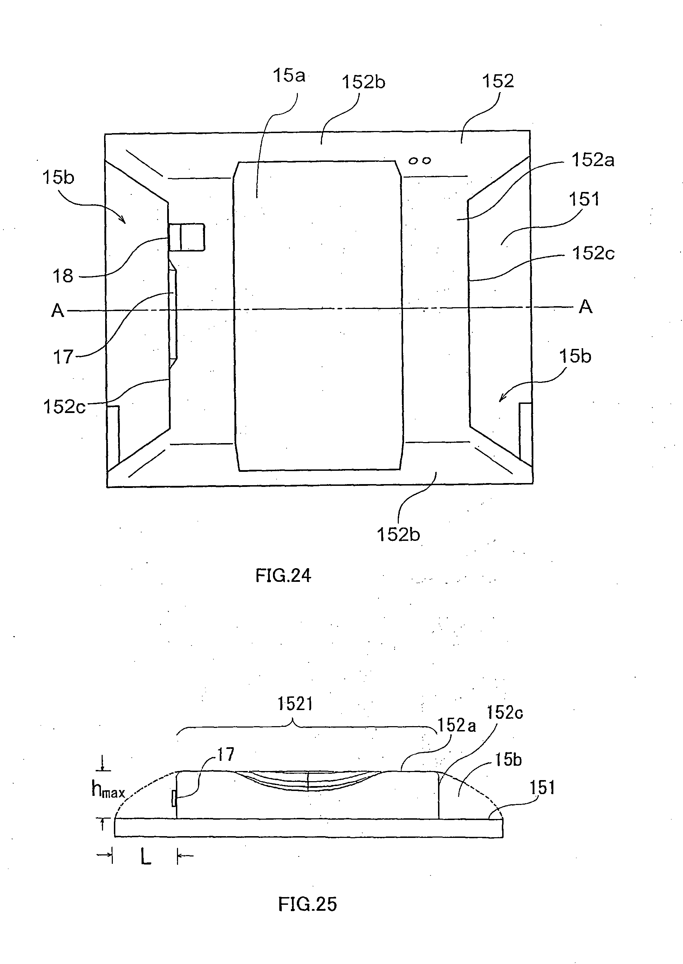

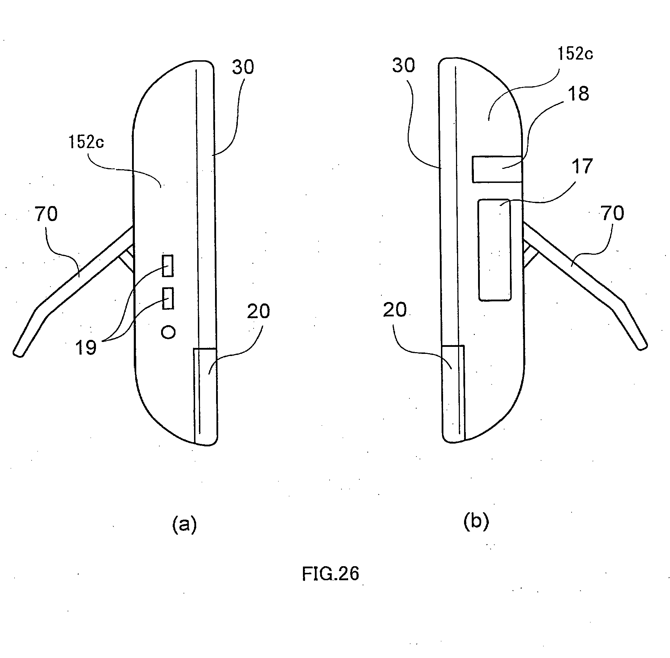

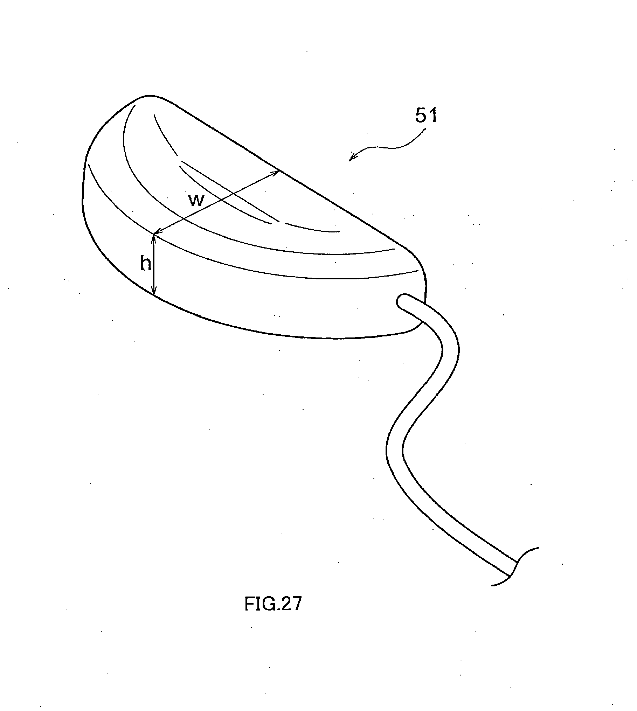

With reference to the drawings from FIG. 24 to FIG. 27, the back side structure will be explained. FIG. 24 illustrates the backside panel and the back side cover of the portable ultrasound imaging apparatus, FIG. 25 illustrates only the essential portion of the cross sectional view of FIG. 24, taken along A-A line, and FIG. 26(a) and FIG. 26(b) illustrate the right side view and the left side view of FIG. 24. FIG. 27 is a perspective view showing one example of the probe connector. Also in FIG. 24, an explanation is provided assuming temporarily that the four orientations; up and down, left and right on the paper respectively correspond to up and down, left and right of the apparatus. However, the orientation of the apparatus may vary freely, and the orientations used for the explanation will not restrict the orientations of the apparatus.

As illustrated in FIG. 24, on the back surface of the main body 10, there is provided the back side cover 152 having a convex shape as a whole relative to the flat back side panel 151, and having a concave portion 15a at the center. In the case where the apparatus is provided with the handle and the stand portion, the concave portion 15a may serve as the space for storing them. The handle and the stand portion are not illustrated in FIG. 24.

The back side cover 152 has a planar section 152a being parallel to the back side panel 151 and the distance from the back side panel 151 being the largest, upper and lower side sections 152b being curved at a tilt from the planar section 152a toward the upper end and the lower end of the main body 10, respectively, and left and right side sections 152c formed, at the position inner than the left and right ends of the main body 10, from the planar section 152a to the back side panel 151. The left and right side sections 152c are curved in the vertical direction toward the upper end and the lower end of the apparatus 10, and joined to the side sections 152b. According to the shape of the back side cover 152, there is formed the space (concave portion 15b) with a trapezoidal shape or a semilunar shape when viewed from the back side, between the side section 152c of the back side cover 152 and each of the left and right ends of the back side panel 151.

As shown in FIG. 27 (b), the port 17 for connecting the connector 51 of the probe 50 is formed substantially in the center (a planar part) of the side section 152c of the back side cover 152 forming the space (the concave portion 15b). This center part corresponds to the narrow width part where the width of the back side cover 152 becomes the narrowest, relative to the width of the main body 10. In the embodiment being illustrated, the port 17 is provided on the side section 152c on the left, out of both side sections, but it may be provided on the right. In proximity to the port 17, there is provided a lock button 18 configured to lock the connection state of the connector 51 that is connected to the port 17. A mechanism to lock the connection state of the connector 51 via the lock button 18 and a release mechanism thereof, are similar to the mechanism of a publicly known ultrasound imaging apparatus. Therefore, an explanation thereof is skipped here.

Here, relations between the space 15b formed by the side section 152c and the back side panel 151, and the probe connector 51 will be explained. As illustrated in FIG. 25, the maximum height *hmax* of the side section 152c (i.e., the maximum thickness of the back side cover 152) is nearly equal to or larger than the thickness h of the probe connector 51 as shown in FIG. 27. The distance L between the side section 152c and the left end or right end of the back side panel 151 is nearly equal to an average width W of the probe connector. Therefore, when the connector 51 is plugged into the port 17 to connect the probe 50 with the apparatus main body 10, the connector 51 is placed substantially in the same plane as the back side surface, and the connector may not protrude considerably from the side surface of the apparatus main body 10. This configuration allows the connector 51 not to project from the main body 10, preventing collision with a structure or a person outside the apparatus, and damage therefrom. Since the connector 51 is placed within the back surface of the apparatus, it is further possible to maintain stable posture, when the main body 10 is placed flatly on a table, without raising up the handle 60 or the stand portion 70.

Since the side surface of the back side cover 152 that delimits the space 15b has a rounded shape being curved (semilunar shape) or a tilted shape (trapezoidal shape), the cable 52 being out from the connector 51 that is connected to the port 17 may be stored partially in the space 15b, and it is guided toward outside along the curved shape or the tilted shape. In addition, when the probe is manipulated on the side of the apparatus, a person or an object may not come into contact with the vicinity of the connector 51 of the cable 52, thereby preventing unreasonable force to be applied to the connection part.

The port 17 may be provided on both of the left and right side sections 152c. Alternatively, the port for the probe is provided on only one side (on the left side when viewed from the back surface), and the other side may be provided with a connecting terminal, e.g., USB port 19 (FIG. 26) for connecting an electronic device, such as a memory, or a power source. In the present embodiment, the port 17 is provided on the left side when viewed from the back surface, and plural USB ports 19 are provided on the right side. With those connection terminals being provided, the main body 10 may be equipped with minimal devices, and if necessary, connection with a memory or an external device may be established, thereby achieving weight saving of the main body and multifunction of the apparatus. On the other hand, if the ports for the probe are provided on both sides, this configuration allows connection to plural probes, or it is further possible to select the port depending on the dominant hand of the operator or the posture during the examination.

In the portable ultrasound imaging apparatus of the present embodiment, when the probe connector 51 is connected to the main body 10, the space 15b is provided, enabling the connector 51 and most of the cable part thereof connected thereto to be placed within the volume of the rectangular parallelepiped defining the tablet shape of the main body 10, thereby achieving stable posture and protection of the connector 51 which is likely to be expensive.

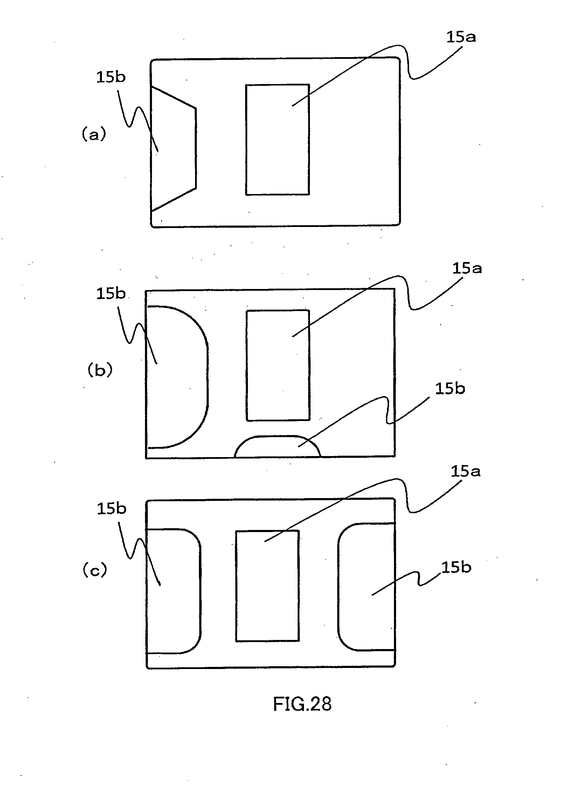

In the embodiment as illustrated, there are provided on both sides of the back side cover, the sections (concave portions 15b) recessed inwardly from the side surfaces of the main body 10, and the port 17 of the connector 51, USB ports, and the like, are provided respectively in those sections. By way of example, as shown in the drawings from FIG. 28(a) to FIG. 28(c), it is possible that only one side is provided with the space, or the shape or the position of the concave portion 15b may change variably.

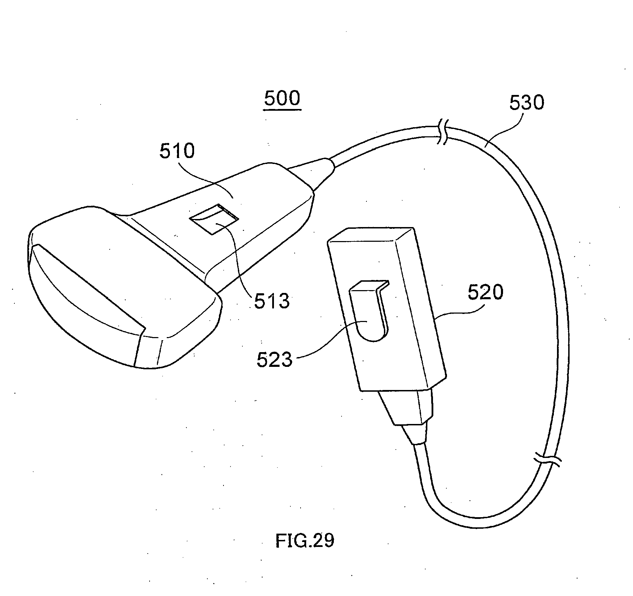

FIG. 29 illustrates another example of the probe that is applicable to the present embodiment. This probe 500 is a wireless-type probe for wirelessly sending and receiving signals to and from the apparatus main body 10, and it is provided with a probe main body 510, an operating portion 520, and a cable 530 for establishing connection between the probe main body 510 and the operating portion 520.

The probe main body 510 has the same configuration as the probe main body 50a of the probe 50 as shown in FIG. 5, and it is made up of one dimensional array or two dimensional arrays of ultrasound transducers (piezoelectric elements), a backing material, and the like, a surface where piezoelectric element arrays are arranged, coming into contact with the subject with pressure, transfers ultrasound waves to the inside of the subject, and receives ultrasound echo signals reflected from the inside of the subject.

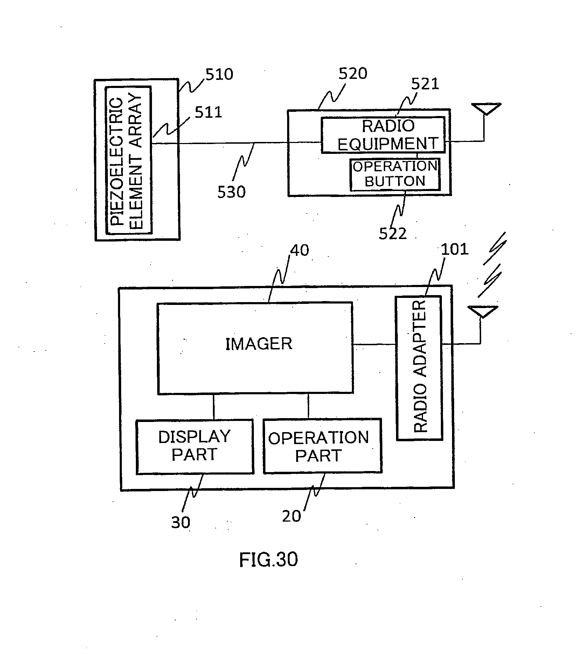

The operating portion 520 has a size allowing the operator to hold it in hand for manipulation, similar to the probe main body 510, and as shown in FIG. 30, it is provided with radio equipment (wireless send and receive means) 521, and the operation button 522. The radio equipment 521 converts the electric signals from the piezoelectric element array 511 in the probe main body 510 and signals from the operation button 522, into electric waves and infrared rays, and further converts the electric waves and infrared rays transferred from the radio adapter (wireless transmit and receive means) on the main body 10 side, into electric signals and outputs the signals to the piezoelectric elements 511.

The radio adapter on the main body 10 side is connected to the port 17 to which the connector 51 of a wired probe is connected, and the radio adapter is stored in the space 15b on the back side of the main body, to which the port 17 faces.

The operation button 522 sends a predetermined command to the ultrasound imager 40, and one or more than one buttons may be provided. The function of the operation button may be redundant with any of the functions of the operation buttons provided on the operation panel 20, or it may have a different function. By way of example, it may be provided with functions for performing a process that is frequently executed during the examination, such as a freeze key for pausing the display screen during the imaging, and a record key for recording and storing the image on the display screen. When the probe main body 510 has a built-in battery, or the like, a switch for the function of on/off may be included.

This probe 500 is further provided with a mechanism to couple the probe main body 510 with the operating portion 520 in a detachable manner. Specifically, as shown in FIG. 29, a hook 523 and a hook receiver 513 to be engaged with each other are formed respectively on the external cases of the operating portion 520 and the probe main body 510. In the illustrated embodiment, the hook receiver 513 is formed at a part that serves as a holder of the probe main body 510, and the hook 523 is formed on one surface of the operating portion 520 having the shape of approximately rectangular parallelepiped. The flat surface of the hook 523 is made to slide to be inserted into the hook receiver 513, thereby coupling both parts. FIG. 31 illustrates the coupled state. In this case, since the probe main body 510 and the operating portion 520 are joined, they may be carried while hooked on a hand or on other equipment as appropriate, or they may be moved along with other equipment, under the condition that the cable 530 is tied together into a loop-like bundle.

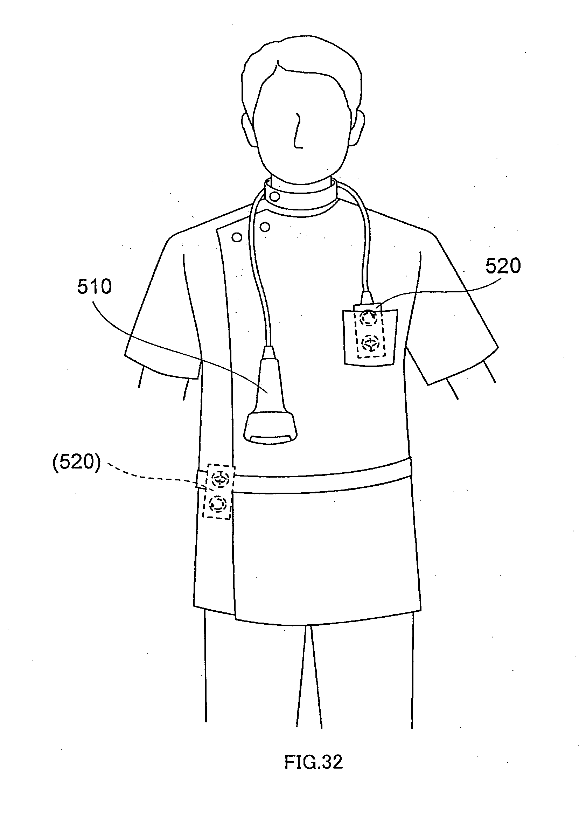

As illustrated in FIG. 32, when an examination is performed, the hook 523 is utilized to hook the operating portion 520 in a pocket or on a belt of the operator, allowing the operator to manipulate the probe main body 510 under this condition. The hook 523 may be a clip type, and in that case, the operating portion may be secured on any position by the clip.

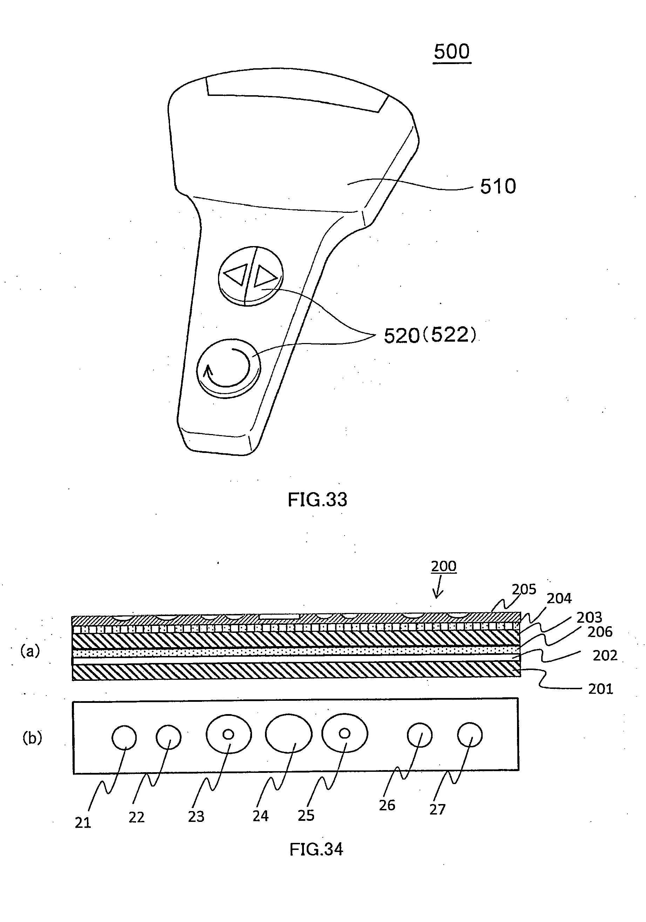

As for the wireless probe 500 as shown in the drawings from FIG. 29 to FIG. 32, the probe main body 510 and the operating portion 520 are connected via the cable 530, but it is possible to eliminate the cable. In this case, as shown in FIG. 33, the probe main body 510 is provided with the operating portion 520 and a wireless transmit and receive means (not illustrated).

Instead of the probe 50 that is connected to the main body 10 via the cable as shown in FIG. 5, the wireless probe 500 as described above may be employed, and this further facilitates carrying the probe and treating the probe during the examination.

### <Multifunctional operation buttons>

Next, an explanation will be provided as to the multifunctional operation buttons being a characteristic of the portable ultrasound imaging apparatus of the present embodiment.

The operation panel 20 of the portable ultrasound imaging apparatus of the present embodiment is a type of touch screen (touch panel), and there are provided operation buttons 21 to 27 that fulfill the button functions according to touch operations by the operator. The touch panel is provided with a pair of electrodes being placed placing a spacer therebetween to sense variation in voltage between both electrodes being caused by the operator's finger coming closer, or variation of resistance caused by the depression by the finger. There are known a capacitance touch panel and a resistive touch panel, and any of those touch screens may be employed. The operation panel 20 of the present embodiment is characterized in that the shape of the operation button is not flat relative to a surface of the touch screen, and it has a predetermined concave shape. The function of the operation button may vary depending on a mode of imaging and a flow of examination, and this variation is one of the other characteristics.

The shape of the button may not be the concave but a convex shape protruding from the touch screen surface, or it is further possible to provide a convex portion partially protruding in the concave shape. As the button shape, the concave or the shape having a partial convex portion within the concave is preferable, in order to prevent malfunctions of the buttons.

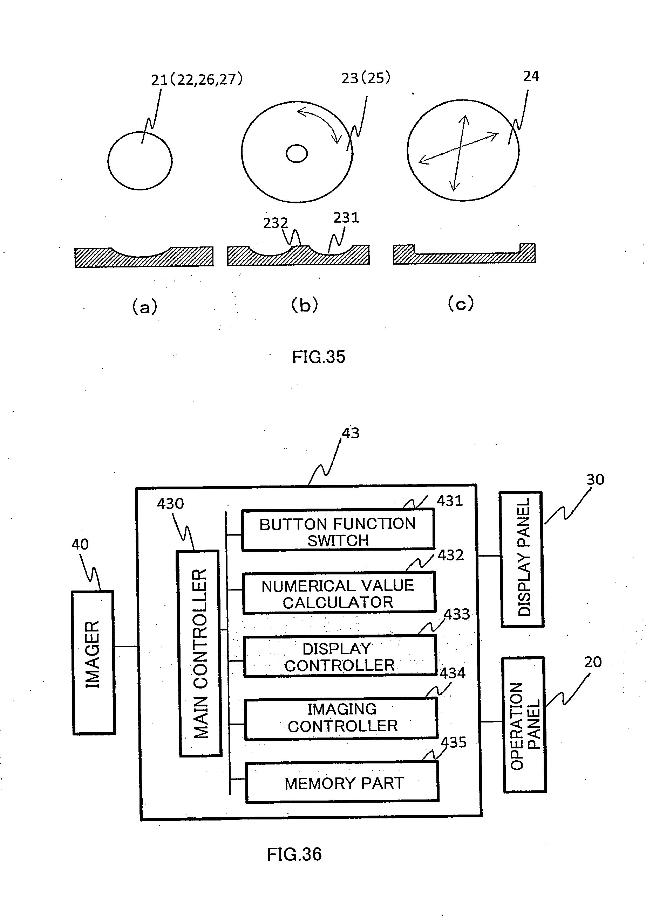

Firstly, with reference to FIG. 34 and FIG. 35, an explanation will be provided as to a characteristic in the shape of the operation button. FIG. 34(a) and FIG. 34(b) schematically illustrate a cross section and a planar form of the operation panel, FIG. 35 (a), FIG. 35(b), and FIG. 35(c) are cross sectional views and top views of the operation buttons 21, 23, and 24 provided on the operation panel of FIG. 34. FIG. 34 illustrates, as an example, a capacitance (projective) touch panel, but the touch panel is not limited to this type.

As shown in FIG. 34(a), the touch panel 200 constituting the operation panel 20 has a structure laminating via an adhesive layer 206, a first layer electrode 202 formed on the first substrate 201, a second layer electrode 204 formed on the second substrate 203, and a cover 205 being an uppermost layer. The first layer electrode 202 and the second layer electrode 204 may be made of a conductive material, such as ITO, for instance, and they are formed as rectangle electrode patterns according to a method such as a sputtering, on the substrates 201 and 203 made of an insulator such as glass and plastic. The cover 205 is also made of an insulator such as plastic, similar to the substrate. The alignment of the rectangles of the first layer electrode 202 and the second layer electrode 204 are orthogonal to each other, and one serves as X-electrode, and the other serves as Y-electrode. When current flows in those electrode patterns, variation in capacitance is sensed, which is generated on the cross point of the electrode patterns, immediately below the position where the operator touches the cover, thereby detecting the operation performed by the operator.

The touch panel 200 of the present embodiment features that the positions on the cover 205 respectively corresponding to the operation buttons 21 to 27, are formed in the concave shape, being lower than the cover surface. With forming the operation buttons in this concave shape, the operator is allowed to know the position where to manipulate, even when he or she faces a patient being the examination target, or when the operator is checking the image, for instance, without turning his or her eyes toward the operation panel 20.

Further in the present embodiment, the concave shape of the operation button varies depending on the function of the button. The functions of the button may include, a function to select a predetermined operation or turn on/off the operation according to depressing action, a function of instructing to change a numerical value according to the rotational manipulation of the finger, and a function of instructing to change the position according to the shifting operation of the finger, and the like. By way of example, the operation buttons 21, 22, 26, and 27 are provided for selecting predetermined actions or inputting of/off only, and it is only required to detect whether or not there is a touch operation on a position where the button is located. This shape is a spherical concave, as illustrated in FIG. 35(a), which allows easy touch and makes dirt hardly built up. The size is not limited, but by way of example, the diameter of the outer periphery is around 10 to 20 mm, and the depth of the concave is around 1 mm.

The operation buttons 23 and 25 have a function of instructing to change numerical values, according to the rotational manipulation by a finger, and it is possible to input a selection of processing (menu), increase or decrease of a numerical value, positional shifting, and the like. As shown in FIG. 35(b), the operation buttons 23 and 25 have a circular outer periphery similar to the operation buttons 21, etc. The diameter thereof is larger than that of the operation buttons 21, etc., and there is space inside the concave part 231, allowing the finger of the operator to move rotationally, with a convex part 232 formed on the center thereof. The size of the operation buttons 23 and 25 is not limited, but by way of example, the diameter is around 25 to 40 mm, the depth of the concave is around 1 mm, and the diameter of the convex part 232 is around 10 to 15 mm. As for the operation buttons 23 and 25 with this shape, the concave space 231 is touched by the finger and the finger performs clockwise or anticlockwise movement, thereby changing the touched point, i.e., the cross point of the electrode patterns. Then, the variation in position of the cross point is detected as variation of a predetermined numerical value, or the like.

The operation button 24 has a function of instructing a shift of the position according to the shifting operation of the finger, i.e. , the track ball (track pad) function, and this operation button has the concave shape, with a flat bottom. FIG. 35(c) illustrates the button with a circular outer periphery, but as far as there is an area that allows the finger touching the cover 205 to perform dragging operations, any shape is applicable. On this operation button 24, the finger shifts in any direction while the finger is touching the flat bottom, thereby allowing the pointer displayed on the screen of the display panel 30 to move to any position on the screen, thereby setting a position or region via the pointer, for instance, setting an ROI or the width of sample gate, measuring the distance, or the like.

Next, with reference to FIG. 36 to FIG. 40, functional variation of the operation button will be explained, being a second characteristic of the operation panel of the present embodiment. In the following explanation, operation buttons provided on the operation panel 20 are taken as an example. It is to be noted, however, that this characteristic is also applicable to the operation button 81 on the handle and the operation button 82 on the main body back side, as explained in the first embodiment.

On the operation panel of a conventional ultrasound imaging apparatus, there are provided plural operation buttons and pointers, each of which is assigned one function. Functions necessary for operating the ultrasound imaging apparatus are diverse, and for example, the imaging mode may include, B-mode, M-mode, D (Doppler) mode, 3D mode, and the like, and there are various conditions to be set for each imaging mode and various processes performed during the imaging. Therefore, in order to ensure operability and arrange such large number of operation buttons, the operation panel has no other choice but to be a large area. In the present embodiment, plural functions are assigned to one operation button, and those functions are switched, thereby providing the operation panel that is suitable for a tablet-type small-sized ultrasound imaging apparatus.

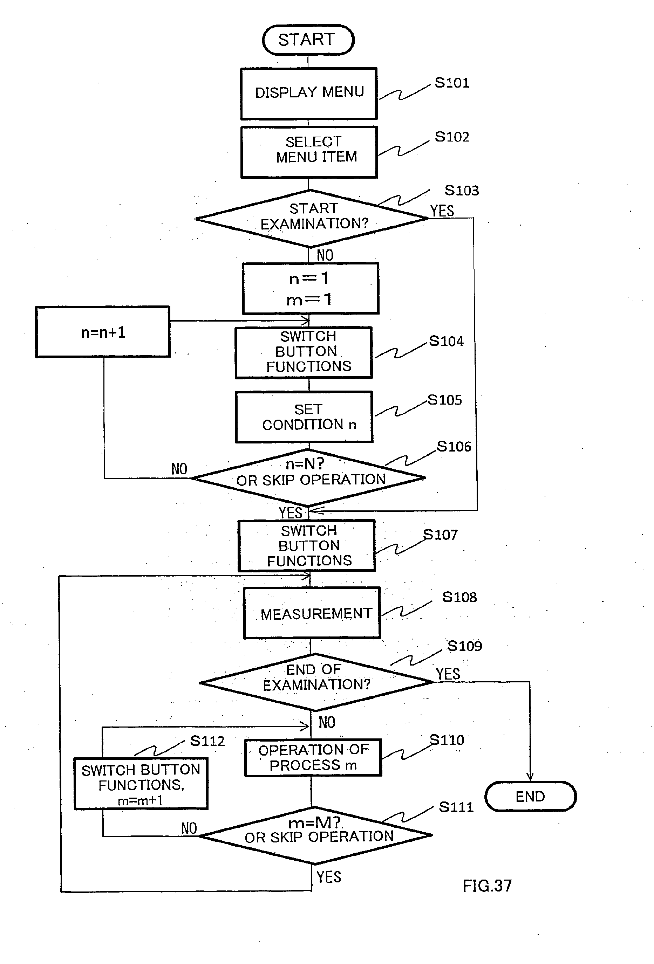

FIG. 36 is a functional block diagram showing functions of the controller 43 that controls the GUI configured by the operation panel and the display panel, and FIG. 37 is a flow diagram showing a control procedure.

As shown in FIG. 36, the controller 43 is provided with a main controller 430, a button function switch 431, a numerical value calculator 432, a display controller 433, and an imaging controller 434.

When a predetermined operation mode is selected according to manipulation on a particular operation button of the operation panel 20, the button function switch 431 switches the functions of other operation buttons along with the selected operation mode. By way of example, the functions are switched in such a manner that plural conditions that are usually set in the predetermined imaging mode are inputted sequentially. The order for inputting the conditions may be configured in advance, or the order may be selected by a user. In the predetermined imaging mode, functions may be switched in such a manner that a general examination routine is sequentially executed. Details of the function switching will be described later.

When the operation button having the numerical value input function (hereinafter, collectively referred to as the operation button B) is manipulated, the numerical value calculator 432 calculates the amount of change of the numerical value such as an imaging condition that is assigned to the operation button B. By way of example, when the operation buttons 23 as shown in FIG. 35 is manipulated, the distance (angle) operated by the finger is calculated according to the change of the coordinate, and it is converted into the amount of change from a preset value of the condition being assigned to the operation buttons 23 when the value was manipulated (e.g. , gain, frequency, and the like). When the operation button having the track ball function (hereinafter, referred to as the operation button C) is manipulated, the position or distance the pointer has moved is calculated. Then, a result of the calculation is transferred to the imaging controller 434 and the display controller 433.

The display controller 433 controls displaying of various-mode images and displaying of GUI. By way of example, a pointer is displayed on the screen, and the pointer is moved along with the operation of the operation button C. In addition, the display controller displays the region set by the operation button C, by a linear diagram indicating the ROI, in a superimposing manner on the image. Further, in response to the operation on the operation button B, the display controller performs magnification change such as scaling of the image, and displays the calculation result by the numerical value calculator 432, in a display region other than the image.

The imaging controller 434 controls each element (ultrasound transmitter and receiver, and the like) of the imager 40 so that the imager performs starting of measurement in the imaging mode being selected, and pausing M-mode tracing or D-mode tracing, referred to as "freeze", in response to the manipulation on the operation button (hereinafter, referred to as the operation button A) having a function of a specific on/off function, or the measurement is performed according to the imaging conditions being set in response to the manipulation on the various operation buttons.

In view of the configuration of the aforementioned controller 43, an explanation will be provided as to the procedure of the controller, focusing on the switching function of the operation buttons, along with the progression of the measurement. FIG. 37 is a flow showing one example of the procedure. It is to be noted that in the following explanation, the function assigned to each operation button is just one example, and the correspondence between the function and the operation button may be changed optionally.

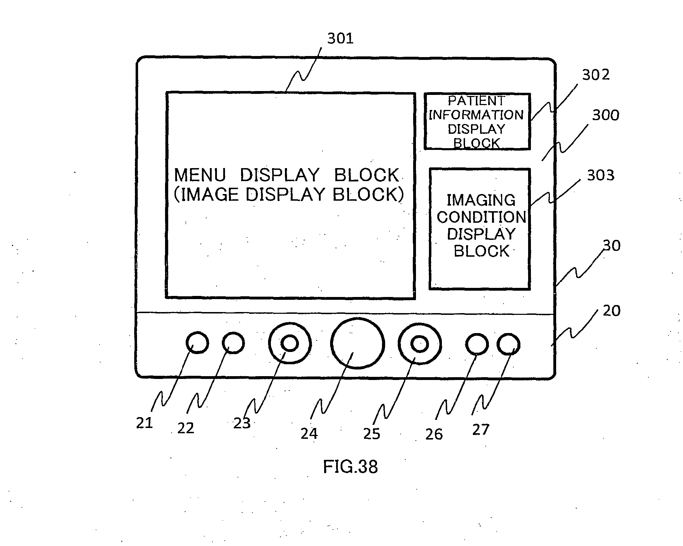

When the power supply of the apparatus is turned on, as illustrated in FIG. 38, there is displayed a menu screen (menu display block 301) including choices of the imaging mode (step S101). In this case, information and previous data regarding the examination target are read from the memory, or the like, connected to a USB port, for instance. Thus read-in patient information, and the like, may be displayed in the patient information display block 302. In the case where a preset value of the imaging condition is determined for each examination region, a menu screen for selecting the examination region may be displayed on the screen. The present value of the imaging condition may be read from an external memory. When the imaging mode is displayed on the menu screen (menu display block 301), the functions are assigned to the operation buttons as the following, for instance; a function of the imaging mode selection button is assigned to one of the operation buttons B (e.g., the operation buttons 23), and a function of finalizing the imaging mode selection is assigned to one of the operation buttons A (e.g., the operation buttons 26).

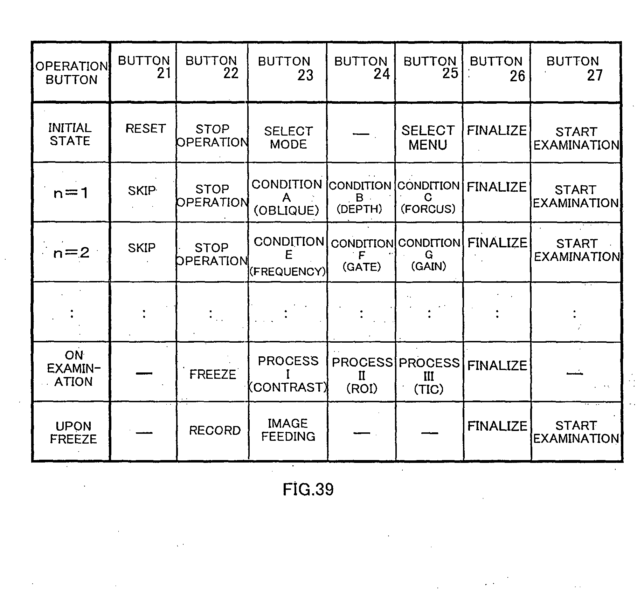

FIG. 39 illustrates one example of the function assignment on the operation buttons provided on the operation panel as shown in FIG. 34. In the example as illustrated, in the initial state, the operation button 21 serves as a "reset button" for the apparatus to resume the previous state, the operation button 22 serves as a "pause" button or "freeze" button to pause the process currently progressing, the operation button 23 serves as a "menu selection button", the operation button 24 serves as a "position designation" button, the operation button 25 serves as a "numerical value designation" button or a "preliminary menu selection" button, the operation button 26 serves as "Enter" button for finalizing the operation, and the operation button 27 serves as an "examination start" button.

In this initial state as described above, firstly, the operation button 23 is manipulated to select any item from the plural items in the menu displayed on the display screen.

In selecting the menu item, when the concave part 231 of the operation buttons 23 is manipulated by the finger, a highlighted item position moves variously along with the movement of the finger, among the plural menu items displayed on the display screen. When a menu item to be selected is highlighted, the movement of the finger is stopped, and the operation button 26 is touched. Then, this finalizes the selecting operation according to the operation button 23, and the menu item being highlighted is selected. The menu item being selected initially is an imaging mode, for instance. The ultrasound imaging apparatus is provided with plural imaging modes; B-mode, M-mode, D-mode (Doppler mode), and the like, and the aforementioned operation selects any of the imaging modes (step S102).

When the B-mode is set as the imaging mode, for instance, the button functions are switched to set the imaging conditions of the B-mode, and on the display screen as shown in FIG. 38, the B-mode imaging conditions are displayed in the imaging condition display block 303. In the menu display block 301, there are displayed an image and a GUI necessary for setting the conditions. The display controller 433 performs this display switching.

The B-mode imaging condition includes; frequency, gain, and the depth, width, oblique angle, focus position, and focus zone position of a target portion to be displayed, and other conditions relating to the displaying operation, such as contrast of the displayed image and noise removal. Some conditions may be set prior to starting the measurement and other conditions may be set after starting the measurement, and when the imaging conditions are preset as described above, the examination starts after modifying the preset values, or without performing the modification. In other words, when the "examination start" button 27 is manipulated after selecting a menu item (S103), the ultrasound beam scanning starts without setting the conditions, and the examination starts. If the examination start button 27 is not manipulated, the button function is switched to the function of setting the conditions for the imaging mode being selected (S104). In order to set plural conditions using a small number of operation buttons, in the embodiment as shown in FIG. 37, the function of the operation button is switched to a function for a different imaging condition, after one imaging condition is finalized. The order of the imaging conditions to be switched is predetermined and stored in the memory part 435, and the button function switch 431 switches the button functions, according to the predetermined order, thereby setting the imaging conditions sequentially.

By way of example, upon setting the initial conditions, it is assumed that the operation button 23, the operation button 24, and the operation button 25 are switched to have the functions of setting the oblique angle, adjusting the position (depth and width), and setting the focusing position, respectively. In this case, when the ring-like part of the operation button 23 is manipulated by the finger, there is displayed a diagram on the display screen (e.g., the block 301 in FIG. 38), showing the oblique angle with respect to the vertical plane, and the angle varies along with the movement of the finger. Specifically, when the operation buttons 23 is rotationally manipulated with touching the concave part 231 thereof by the finger, in clockwise or in anticlockwise, the numerical value calculator 432 calculates positional change, and further calculates the amount of angular change in association with the amount of positional change. The display controller 433 creates a linear diagram representing the oblique angle, in accordance with the amount of angular change, calculated by the numerical value calculator 432, and display the linear diagram on the display panel, in such a manner as superimposed on the vertical image. The angle being calculated may be displayed in the form of a numerical value. The linear diagram representing the oblique angle or the numerical value of the angle continues to vary, as far as the manipulation of the operation buttons 23 continues, and if the moving direction of the finger is turned to be opposite, the variation of the angle is also inverted. When a desired oblique angle is displayed, the operator touches the operation buttons 26, thereby finalizing the oblique angle.

In addition, when the operation buttons 24 is manipulated, the pointer is placed at a desired position, and upon touching the operation buttons 26, a start point in the X-direction (width) or in the Y-direction (depth) is determined. Subsequently, on the operation buttons 24, dragging operation is performed to move the point by desired distance in the X-direction or in the Y-direction, and upon touching the operation button 27 at a halting point, the end point in the X-direction or in the Y-direction is determined. With those operations as described above, it is possible to finalize the depth and the width of the target portion that is displayed in the B-mode.

The operations above are similar in setting the focus, but setting the focus may include plural processing such as setting a position or a focus zone, and it is also possible to change the functions of the operation buttons hierarchically, so that the setting is performed by combining the operation buttons 23 to 25.

Also in the case of setting a condition other than the aforementioned conditions, when setting of one condition is finished, the button is made operable after switching the function used for setting that previous condition, to set the next condition (S105, S106, and S104). It is to be noted that one of the operation buttons, for example, the operation button 22 is assigned a skip function for skipping the predetermined order of condition setting, so that setting or modification may be skipped as to the conditions that the operator does not need to change (the operation is executable under the default conditions).

When setting of necessary conditions is completed, the functions of the operation buttons are switched so that according to each operation button, following processing is executed during the examination, such as changing the conditions during the examination, adjusting the contrast, setting an ROI and sample region, and TIC analysis (S107). As illustrated in FIG. 39, for instance, the function for adjusting the variation of numerical values such as the contrast, is assigned to the operation button 23 and the operation button 26 (Enter button) for finalizing the adjustment, the function for instructing the processing that needs positional designation, such as setting the ROI or the sample region, is assigned to the operation buttons 24, and the function for instructing to start a predetermined processing such as the TIC analysis is assigned to the operation buttons 25 and the operation buttons 26 (Enter button). Modification of the conditions during the examination may be assigned to the operation buttons 21, for instance, and it is further possible to configure such that when this operation button is manipulated, the process returns to the processing loop (S103 to S106) for setting the conditions.

After switching the button functions in the step S107, when the operation button 27 for the "examination start" is manipulated, the measurement is started, and the display controller 433 displays a B-mode image on the display screen (e.g., display block 301). In the middle of the examination, when the operation button which is assigned the function for instructing a predetermined process is manipulated, this process starts (S109 and S110). If the number of processing during the measurement, including the condition setting for displaying, and the like, is not able to be covered by the number of operation buttons provided on the operation panel 20, the button functions are switched as explained in the step S110 to S112. Also in this case, the processing is performed according to a predetermined order and this is similar to the step S105 for setting the conditions. Furthermore, a specific operation button is assigned the skip function to skip unnecessary processing.

In the initial imaging mode selection (S102), when the M-mode or the D-mode is selected, the conditions being set and the details of the processing performed during the measurement may be different, but the examination proceeds, with switching the functions of the operation buttons, basically according to the procedure as shown in FIG. 37. In the M-mode or the D-mode, the image displayed during the examination is frequently paused, and processing for recording (freezing) the image is performed as required. For this kind of highly frequent processing, one or two operation buttons are assigned the functions for instructing "freeze", "record", and the like, during the time of examination, enabling the "freeze" or "record" operation to be performed as needed. As illustrated in FIG. 39, for instance, the operation button A (e.g., the operation buttons 22) is assigned the freeze function. When the apparatus becomes in the state of "freeze" according to the manipulation of this operation buttons 22, the operation button B (e.g., the operation buttons 23) is provided with a function for sequentially displaying the images before the freeze state, and simultaneously the function of the operation buttons 22 is switched from the function for instructing to perform "freeze", to the function for instructing to perform "record".

By switching the button functions according to the processing flow and procedure, the operator is allowed to proceed with the examination smoothly. Instructions such as pausing and finalizing the operation, and starting the examination are assigned to identical operation buttons, whereby the operator is allowed to proceed with the examination without hesitation, even when the button function varies.

In the embodiment as shown in FIG. 37, it is described that the button functions are sequentially switched in setting plural conditions n, or plural processing and manipulations, and switching of the button functions for this case may include switching not only one operation button but also plural operation buttons simultaneously. In other words, there is a possibility that functions of some operation buttons are switched, but the functions of some other operation buttons are not modified.

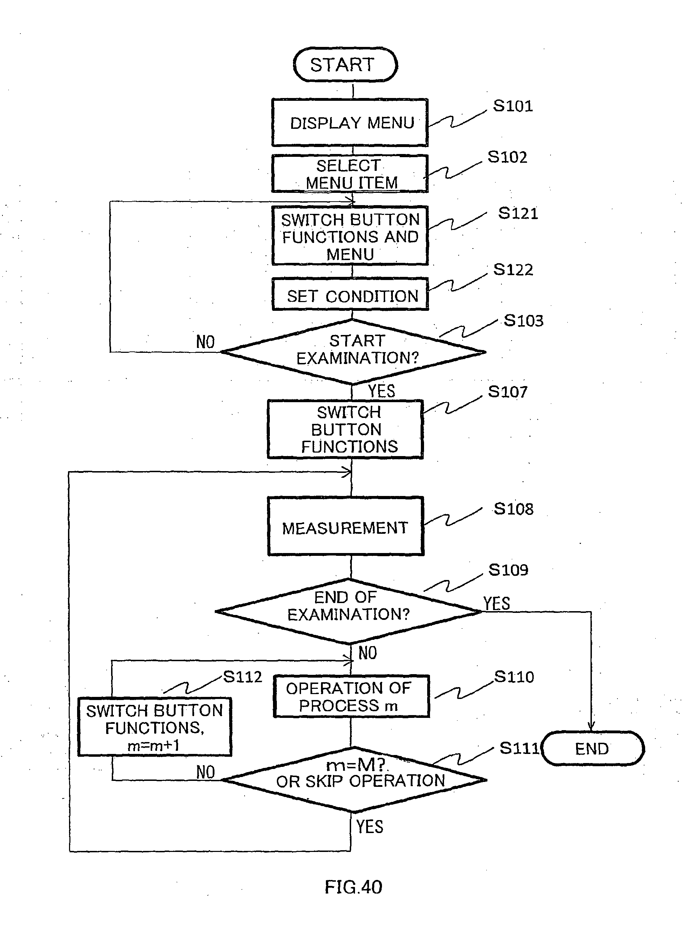

In the example as shown in FIG. 37, it is explained that the conditions and the order of the processes are determined in advance, but it is alternatively possible to display plural conditions as menu items as shown in FIG. 40, without determining the order, and a condition is selected from the menu items for the condition setting. For this case, when one item is selected on the menu screen for selecting the imaging mode, for instance, the display controller 433 switches the screen to another screen displaying conditions that are to be set in the selected imaging mode (S121). On this screen, the imaging conditions associated with the selected imaging mode (e.g., the B-mode) are displayed as the menu items, and thus according to the manipulation of the operation button B or the operation button C (e.g. , the operation button 24), a condition to be set is selected on the screen where plural conditions are displayed. For selecting a desired condition, for instance, the operation buttons 24 is manipulated to move the position of the pointer on the screen, to the block indicating the condition (e.g., frequency) to be set, and then it is finalized by the Enter button 26. According to this operation, a condition to be set (e.g., any of the depth, oblique angle, gain, and the like) is selected.

Next, setting of thus selected condition is performed (S122). The condition is set, by manipulating the operation buttons 23, for instance, in such a manner as touching the concave part 231 thereof by the finger, and turning the button in clockwise or anticlockwise. The numerical value calculator 432 calculates the change of positions, and further calculates a numerical value of frequency in association with the amount of positional change. Then, the display controller 433 displays the numerical value of frequency in the condition display block 303 (FIG. 38). When the operator touches the operation button 26 at the time when a desired numerical value is displayed, that finalizes the frequency.

When one condition is set, the process returns to the step S121, and the button functions are switched to prepare for selecting another condition block from the condition menu selection screen, until the examination start button is manipulated (S103). The steps S121 and S122 are repeated until setting of the necessary conditions is completed. When the examination start button is manipulated, the button function is further switched to proceed with the measurement. This procedure corresponds to the procedure from the steps S107 to S112 as shown in FIG. 37.

The portable ultrasound imaging apparatus of the present embodiment is configured such that the operation button is formed as a touch-panel type, and the function of the operation button is switched in response to the examination procedure or the imaging mode, whereby only small number of operation buttons enables implementation of various imaging condition setting and various processes, within a restricted area that is caused by the tablet type apparatus.

According to the portable ultrasound imaging apparatus of the present embodiment, the shape of the operation button is configured as a concave form or a convex form, relative to the operation surface, thereby allowing the operator to manipulate the operation panel with reliability, even while paying attention to the patient or the image shown on the display panel. In particular, the shape of the button varies depending on the function of the operation button, thereby implementing various functions and enhancing the reliability in operation.

It is to be noted here that the operation buttons as shown in FIG. 34 and FIG. 35 are just examples, and the types, the number, or the layout of the operation buttons may be changed as appropriate. In addition, the assignment of the functions to the operation buttons as shown in FIG. 39 is also an example, and it may be modified depending on the examination target portion and the imaging mode.

### <Holding structure of the probe>

The portable ultrasound imaging apparatus of the present embodiment is characterized in that a probe holder is provided on the back surface of the main body. The probe holder may be mounted detachably on a support structure of the handle and/or the stand portion, for instance.

During the examination, the probe is placed on a portion of a patient, or the like, to perform examination, and when the examination is suspended or upon carrying the apparatus, it is necessary to store the probe in a holder. The portable ultrasound imaging apparatus of the present embodiment is provided with a probe support structure (probe holder), by utilizing the support structure of the handle 60 and the stand portion 70 on the back surface of the main body 10.

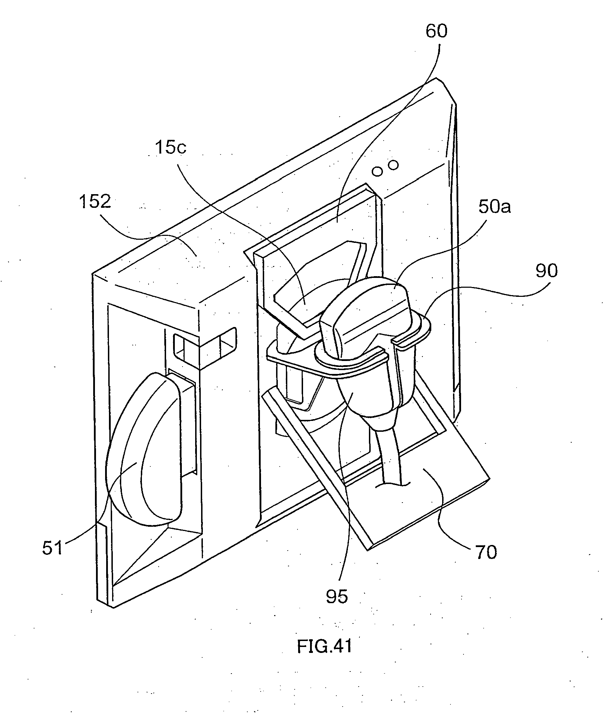

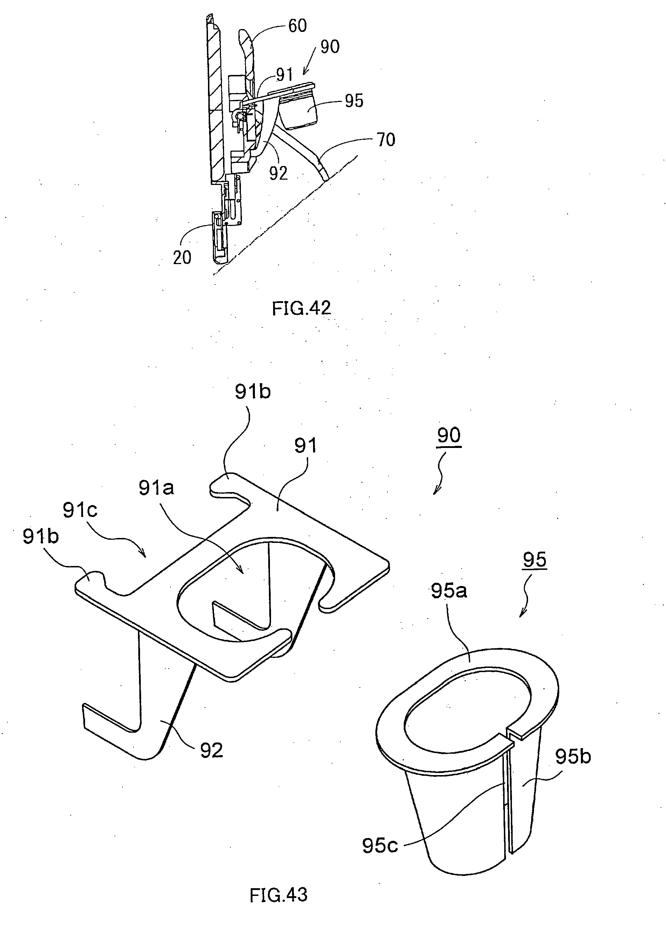

Hereinafter, with reference to the drawings from FIG. 41 to FIG. 43, details of the probe holder will be explained. FIG. 41 illustrates the back surface of the main body viewed from the back side in perspective, FIG. 42 is a cross-sectional view, and FIG. 43 illustrate the probe holder and the cup in perspective, when the cup is removed.

Those figures illustrate that both the handle 60 and the stand portion 70 are provided, and the probe holder is applied to the apparatus having the same configuration as the apparatus of FIG. 17. The feature of the present embodiment is that the probe holder is provided on the back surface. Therefore, both the handle 60 and the stand portion 70 are not necessarily provided, and only either one of the handle 60 and the stand portion 70 may be provided.

As illustrated, the handle 60 and the stand portion 70 are foldably and rotatably mounted via the support structure in the concave portion 15a of the back side cover 152 provided on the back surface, and the cover 15c covers the support structure. In the embodiment as shown in FIG. 17, the cover 15c has the circular shape, but in here, the cover 15c may have the shape with the left and right sides being cut off, and the probe holder 90 is mounted in such a manner as pinching both sides of the cover 15c.

As illustrated in FIG. 43, the probe holder 90 is made up of the cup supporter 91 and two legs 92 connected to the reverse side thereof, and they may be made of plastic, metal, or the like, and prepared by integrally molded, for instance.

The cup supporter 91 is made up of a flat member having an opening 91a into which the cup 95 fits, and the opening 91a of the cup supporter 91 is partially opened, and the inner periphery forms C-shape. In addition, a notch 91c having a substantially rectangular shape is formed on the cup supporter 91 on the other side of the opening 91a, provided back-to-back with the opening 91a, and as a result, a pair of bent portions 91b are formed making the notch 91c therebetween. The size in the longitudinal direction of the notch being substantially rectangle in shape, approximately coincides with the width in the left and right direction of the cover 15 having the shape with the left and right sides being cut off. The distance between the tips of the pair of the bent portions 91b is made a little narrower than the width of the cover 15c, and accordingly, as shown in FIG. 42, the bent portions 91b is fixed on the cover 15c in such a manner as placing the cover 15c therebetween.

The legs 92 extend substantially in the perpendicular direction relative to the support surface of the cup supporter 91, and the tips 92a thereof are bent, being shaped like a letter L. The cover 15c covering the support structure of the handle 60, and the like, has the concave parts being formed, respectively in association with the ends of the legs 92, and when the bent portions 91b of the cup supporter 91 are mounted on the cover 15c, the tips 92a of the legs 92 are structured to be engaged with the concave parts formed on the cover 15c, to support the probe holder 90 together with the bent portions 91b.

The cover 15c may be provided with a portion (narrowed width portion) whose width in the left-right direction is narrower than the distance between the tips of the pair of bent portions 91b, and in this case, the narrowed width portion is utilized to render the probe holder 90 to be detachable. By way of example, after the bent portions 91b of the probe holder 90 are inserted, the probe holder is made to slide in the vertical direction, from the narrowed width portion of the cover 15c, to the position where the tips 92a of the legs 92 of the probe holder 90 are engaged in the concave portions of the cover 15c, the tips 92a are pressed into the concave parts of the cover 15c, and accordingly the probe holder is mounted. When the probe holder 90 is removed from the cover 15c, this procedure is performed the other way around.

As shown in FIG. 43, the cup 95 that is fixed on the probe holder 90, is made up of integrally formed flange portion 95a and cylindrical portion 95b continuous thereto, and it is similar to the cup that is used in a general probe holder. The cylindrical portion 95b has a diameter that becomes gradually smaller, from the flange portion 95a to the end. The flange portion 95a and the cylindrical portion 95b are provided with one slit 95c along the axis of the cylinder, and this configuration facilitates putting the probe 50a in and out, the probe being connected to the cable 52 as shown in FIG. 5. The opening 91a of the cup supporter 91 has the inner diameter substantially equal to the outer diameter of the cylindrical portion 95b of the cup 95, and by inserting the cup 95 from above the opening 91, the flange portion 95a is put on the support surface of the cup supporter 91, achieving a support by the cup supporter 91. In this case, the opened part of the opening 91a of the cup supporter 91 coincides with the slit 95c of the cup 95.

The portable ultrasound imaging apparatus of the present embodiment is provided with the structure for supporting the probe holder on the back side of the apparatus, and therefore, it is not necessary to prepare the probe holder, separated from the main body, thereby enhancing the convenience of the portable apparatus.

The probe holder of the present embodiment is mounted, utilizing the space between the handle 60 and stand portion 70, and it does not interfere with the functions of the handle 60 and the stand portion 70. In other words, even when the probe holder 90 is mounted, it is possible to raise the handle 60 for manipulation, as far as the probe is not placed on the probe holder, and further pull out the stand portion 70 to make the apparatus to stand on the table. Under this condition, as shown in FIG. 41, it is possible to put the probe 50 in the probe holder 90, or take out the probe 50 therefrom.

As described so far, the embodiment of the portable ultrasound imaging apparatus of the present invention has been explained as to each of the features. The scope of the present invention is described in the claims, and specific structures, shape, layout, and the number of each element may be modified arbitrarily, within this scope of the invention. In addition, some of the aforementioned features are not absolutely necessary in the present invention, and some features may be omitted.

### Industrial Applicability

According to the present invention, the portable ultrasound imaging apparatus is provided which allows one hand to hold the apparatus, and the other hand to manipulate the probe, and also allows manipulation via the operating portion provided on the back side, achieving excellent ease of operation during the examination. This portable ultrasound imaging apparatus facilitates examination, not only in a laboratory, but also in a site with spatial restriction such as a hospital room, a vehicle, and a place of a house visit by a doctor.

### Explanation of References

10 ... main body, 11 ... front panel, 15 ... back surface of the main body, 15a ... concave portion, 17 ... probe port, 20 ... operation panel, 21 to 27 ... operation buttons, 28, 29 ... operation buttons (back surface), 30 ... display panel, 40 ... ultrasound imager, 43 ... controller, 431 ... button function switch, 432 ... numerical value calculator, 433 ... display controller, 434 ... imaging controller, 47 ... input part, 50 ... probe, 51 ... connector, 60 ... handle, 70 ... stand portion, 81, 82 ... operation buttons (operating portion), 90 ... probe holder, 100 ... ultrasound imaging apparatus, 151 ... back side panel, 152 ... back side cover

## Claims

1. A portable ultrasonic imaging device comprising,
a main body incorporating an electronic circuit constituting an ultrasound imager,
a display panel configured to display an image on a front surface, when assuming one surface of the main body as the front surface and assuming a surface on the other side of the front surface as a back surface, and
an operating portion on the back surface.

2. The portable ultrasonic imaging device according to claim 1, wherein,
a handle is provided on the back surface, and the operating portion is provided on the handle.

3. The portable ultrasonic imaging device according to claim 1, wherein,
a handle is provided on the back surface, and the operating portion is provided on the back surface of the main body, in proximity to the handle.

4. The portable ultrasonic imaging device according to claim 1, wherein,
the operating portion includes an operation button configured to instruct a start and an end of operation, or finalization of processing.

5. The portable ultrasonic imaging device according to claim 1, wherein,
the operating portion includes an operation button having a trackball function.

6. The portable ultrasonic imaging device according to claim 2, wherein,
the operating portion is provided in a concave portion placed on the handle.

7. The portable ultrasonic imaging device according to claim 3, wherein,
the operating portion is provided in a concave portion placed on the back surface.

8. The portable ultrasonic imaging device according to claim 1, comprising,
either or both of a handle and a stand portion being supported on the back surface, in such a manner as rotatable and foldable with respect to the main body.

9. The portable ultrasonic imaging device according to claim 8, comprising the main body comprises a support mechanism configured to support either or both of the handle and the stand portion, wherein,
the support mechanism comprises a rotary plate rotatably mounted on the main body, and a hinge part fixed on the rotary plate, and
either or both of the handle and the stand portion are fixed on the rotary plate via the hinge part.

10. The portable ultrasonic imaging device according to claim 8, wherein,
a concave portion configured to store either or both of the handle and the stand portion, is formed on the back surface of the main body.

11. The portable ultrasonic imaging device according to claim 8, comprising a probe holder in proximity to either or both of the handle and the stand portion.

12. The portable ultrasonic imaging device according to claim 1, comprising an operation panel in parallel with the display panel, on the front surface of the main body.

13. The portable ultrasonic imaging device according to claim 12, wherein,
both the operation panel, and the operating portion on the back surface, serve as an input unit of the ultrasound imager.
